(19)

Europäisches
Patentamt
European
Patent Office
Office européen
des brevets

(11) **EP 2 308 473 A1**

(12) **EUROPEAN PATENT APPLICATION**
published in accordance with Art. 153(4) EPC

(43) Date of publication:
**13.04.2011 Bulletin 2011/15**

(21) Application number: **09773510.4**

(22) Date of filing: **01.07.2009**

(51) Int Cl.:
*A61K 9/16* (2006.01)          *A61K 31/137* (2006.01)
*A61K 31/4045* (2006.01)      *A61K 31/715* (2006.01)
*A61K 38/00* (2006.01)        *A61K 38/28* (2006.01)
*A61K 39/00* (2006.01)        *A61K 45/00* (2006.01)
*A61K 47/30* (2006.01)        *A61K 47/32* (2006.01)
*A61K 47/36* (2006.01)        *A61K 47/42* (2006.01)
*A61K 48/00* (2006.01)        *A61P 3/10* (2006.01)
*A61P 11/10* (2006.01)        *A61P 25/06* (2006.01)

(86) International application number:
**PCT/JP2009/062053**

(87) International publication number:
**WO 2010/001932 (07.01.2010 Gazette 2010/01)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **01.07.2008 JP 2008172669**

(71) Applicant: **Nitto Denko Corporation
Ibaraki-shi
Osaka 567-8680 (JP)**

(72) Inventors:
• **OKUBO, Katsuyuki
Ibaraki-shi
Osaka 567-8680 (JP)**
• **KITAURA, Chieko
Ibaraki-shi
Osaka 567-8680 (JP)**

• **MINOMI, Kenjiro
Ibaraki-shi
Osaka 567-8680 (JP)**
• **PEARSON, Elizabeth
Nottingham NG72RD (GB)**
• **ROBERTS, Clive J.
Nottingham NG72RD (GB)**
• **DAVIES, Martyn C.
Nottingham NG72RD (GB)**
• **STOLNIK-TRENKIC, Snjezana
Nottingham NG72RD (GB)**
• **ILLUM, Lisbeth
Nottingham NG71BA (GB)**

(74) Representative: **Duckworth, Timothy John et al
J.A. Kemp & Co.
14 South Square
Gray's Inn
London WC1R 5JJ (GB)**

(54) **PHARMACEUTICAL COMPOSITION CONTAINING SURFACE-COATED MICROPARTICLES**

(57)     It is an objective of the present invention to provide a pharmaceutical composition that can be used for an efficient administration of low-molecular weight drugs and polymeric compounds such as peptides and proteins by methods other than injection, and a method for producing the composition.

The above-mentioned problem is solved by a pharmaceutical composition for transmucosal administration, comprising (a) a drug having a positive or negative charge at a predetermined pH, (b) a pharmaceutically acceptable small particle and (c) a pharmaceutically acceptable surface-coating polymer capable of being electrically charged at the pH, wherein the surface of the small particle is coated by the surface-coating polymer, the drug is immobilized on the surface of the small particle via the surface-coating polymer, and a complex is formed by a noncovalent interaction between the small particle and the surface-coating polymer and a concurrent electrostatic interaction between the surface-coating polymer and the drug.

## Description

**Technical Field**

The present invention relates to a pharmaceutical composition for transmucosal administration and a production method thereof. More specifically, the present invention relates to a novel pharmaceutical composition for transmucosal administration, comprising a complex consisting of a drug, a small particle and a surface-coating polymer, wherein the surface of the small particle is coated by the surface-coating polymer, the drug is immobilized on the surface of the small particle via the surface-coating polymer, and the complex is formed by a noncovalent interaction between the small particle and the surface-coating polymer and a concurrent electrostatic interaction between the surface-coating polymer and the drug; and a production method thereof.

**Background Art**

The advances of biotechnology have resulted in the discovery of a large number of therapeutic compounds such as peptides, proteins, polysaccharides, polynucleic acids, siRNAs, RNAs, antibodies, antigens, low-molecular weight drugs and the like. However, many of these compounds are difficult to administer into the body by methods other than injection due to their physicochemical characteristics (e.g., large molecular weight, hydrophilicity, instability and the like). For some of these compounds, a multiple daily dosing by injection is necessary. A particular problem, however, is that younger patients do not necessarily comply with this regime of dosing of the compounds (non-patent documents 1 and 2).

When administered via the oral route or via other mucosal routes of delivery across the mucosa such as that in lung, mouth cavity, vagina, nose and the like, these drugs are not easily absorbed due to their physical size and hydrophilicity. Furthermore, these drugs are prone to degradation by enzymes such as peptidases and proteases, which are especially a problem in the gastrointestinal tract. In order to improve the transport of these drugs across mucosal surfaces, formulations containing absorption enhancers have been used with some success especially in delivery by the transnasal route and by the pulmonary route. However, there is a demand for the development of effective methods and compositions to achieve the transport of compounds having a higher molecular weight across mucosal surfaces.

Systems using small particles such as nanoparticles have been widely studied as means for transport of polymeric drugs such as peptides and proteins across mucosal surfaces (non-patent documents 3-5). In the case of peptide and protein drugs, it has been suggested that their stability is low unless the drug is encapsulated into the matrix of a nanoparticle. However, encapsulation of these compounds into nanoparticles is difficult due to the large size of these compounds and the normally hydrophobic environment in the matrix of a nanoparticle and this generally results in a very low loading capacity and hence the need for administration of large quantities of nanoparticles to the mucosal surface. Furthermore, it has been clarified in a publication that transport of nanoparticles across the mucosa is not easily achievable (non-patent document 6).

Prior Art References

non-patent documents

non-patent document 1: Drug Discovery Today, Vol.7, pp.1184-1189 (2002)
non-patent document 2: J. Control. Rel., Vol.87, pp.187-198 (2003)
non-patent document 3: J. Pharm. Sci., Vol.96, pp.473-483 (2007)
non-patent document 4: Biomaterials, Vol.23, pp.3193-3201 (2002)
non-patent document 5: Int. J. Pharm., Vol.342, pp.240-249 (2007)
non-patent document 6: J. Pharm. Sci., Vol.96, pp.473-483 (2007)

**Summary of the Invention**

**Problems to be Solved by the Invention**

It is an objective of the present invention to provide a pharmaceutical composition that can be used for an efficient administration of low-molecular weight drugs and polymeric compounds such as peptides and proteins by methods other than injection. More specifically, it is an objective of the present invention to provide a pharmaceutical composition comprising small particles for the efficient administration of low-molecular weight drugs and polymeric drugs such as peptides and proteins by a route across the mucosa such as that in nose and the like, wherein the composition has superior drug loading efficiency and loading capacity compared to conventional small particle preparations for transmucosal administration, and has achieved improved drug stability. It is also an objective of the present invention to provide a method for the production of the pharmaceutical composition.

**Means of Solving the Problems**

The present inventors focused on transmucosal administration using a small particle system such as nanoparticle as a method for efficiently administering drugs (e.g., peptide, protein, DNA, RNA, siRNA, polysaccharide, antibody, antigen, low-molecular weight compound and the like) by methods other than injection, and conducted diligent investigations. As a result, the present inventors found that drug stability was markedly improved compared to solution preparations of the same drug by preparing a composition comprising a complex wherein a drug-surface-coating polymer complex, which was formed by an electrostatic interaction between a drug and a surface-coating polymer (i.e., a polymer that attaches to the surface of small particles), was immobilized on the surface of a small particle by a noncovalent interaction between the small particle and the surface-coating polymer. Furthermore, the present inventors found that the composition had a superior drug loading capacity compared to small particle preparations of the type wherein a drug is encapsulated. Based on these findings, the present inventors found that a drug delivery system superior to conventional methods can be achieved by using the composition, which resulted in the completion of the present invention. Accordingly, the present invention is as follows.

[1] A pharmaceutical composition for transmucosal administration, comprising (a) a drug having a positive or negative charge at a predetermined pH, (b) a pharmaceutically acceptable small particle and (c) a pharmaceutically acceptable surface-coating polymer capable of being electrically charged at the pH, wherein the surface of the small particle is coated by the surface-coating polymer, the drug is immobilized on the surface of the small particle via the surface-coating polymer, and a complex is formed by a noncovalent interaction between the small particle and the surface-coating polymer and a concurrent electrostatic interaction between the surface-coating polymer and the drug.
[2] The compositions of [1] above, wherein the noncovalent interaction between the small particle and the surface-coating polymer is electrostatic interaction.
[3] The composition of [1] or [2] above, wherein the predetermined pH is the physiological pH of an administration site.
[4] The composition of any one of [1] to [3] above, wherein the drug is selected from the group consisting of peptide, protein, DNA, RNA, siRNA, polysaccharide, antigen and low-molecular weight drug.
[5] The composition of any one of [1] to [4] above, wherein the drug is a drug capable of producing medicinal or vaccine effect.
[6] The composition of [4] above, wherein the drug is insulin.
[7] The composition of [4] above, wherein the drug is at least one drug selected from the group consisting of bromhexine, zolmitriptan and salts thereof.
[8] The composition of any one of [1] to [7] above, wherein the surface-coating polymer is by itself slightly water-soluble at the predetermined pH.
[9] The composition of any one of [1] to [8] above, wherein the surface-coating polymer is at least one polymer selected from the group consisting of chitosan, polyarginine, polyacrylic acid, poly-gamma-glutamic acid and salts thereof.
[10] The composition of any one of [1] to [9] above, wherein the surface-coating polymer is mucoadhesive and/or acts as a transmucosal absorption promoter.
[11] The composition of any one of [1] to [10] above, wherein the small particle comprises a polymer having a carboxylic group or an amino group.
[12] The composition of any one of [1] to [11] above, wherein the small particle is comprised of a poly(lactic acid-glycol acid) copolymer.
[13] The composition of any one of [1] to [12] above, wherein the mean particle size of the complex at the predetermined pH is not less than 10 nm and not more than 50 $\mu$m.
[14] A production method of the composition of [8] above, comprising

(a) mixing the drug, the small particle and the surface-coating polymer at a pH at which the surface-coating polymer is readily water-soluble, and
(b) adjusting the pH of the mixture to the predetermined pH.

[15] A production method of the composition of any one of [1] to [13] above, comprising

(a) mixing the drug, the surface-coating polymer and the small particle under a pH condition under which the drug and the surface-coating polymer have the same sign of the charge, and then
(b) adjusting the pH of the mixture to a pH at which the sign of the charge of the drug changes to the opposite sign, and wherein the drug is an amphoteric drug.

[16] The production method of [15] above, wherein the small particle has a charge of the sign opposite to that of the charge of the drug and the charge of the surface-coating polymer under the pH condition of step (a).
[17] A production method of the composition of any one of [1] to [13] above, comprising

(a) adding dropwise an organic solvent solution of a material of the small particle into an aqueous solution of the surface-coating polymer,
(b) evaporating the organic solvent,
(c) adding the drug and stirring the mixture, and
(d) adjusting the pH of the mixture to the predetermined pH.

**Effect of the Invention**

The use of the composition of the present invention enables an efficient transmucosal administration of low-molecular weight drugs and polymeric drugs such as peptides and proteins, which have so far been difficult to administer by a method other than injection. The drug contained in the composition of the present invention forms a complex with a surface-coating polymer of opposite charge and a small particle and thereby has higher stability (e.g., stability against enzymes, preservation stability) than when contained in a solution preparation, as well as higher drug loading capacity compared to small particle preparations wherein a drug is encapsulated in a matrix of the small particle. Furthermore, it is possible to achieve sustained release or immediate release of the drug and to control transmucosal absorbability of the drug dependent on the type of surface-coating polymer that forms a complex with the drug on the surface of the small particle.

**BRIEF DESCRIPTION OF THE DRAWINGS**

Fig. 1 explains the mechanism of the production of surface-coated small particles (surface carrier system) comprising PLGA core particles surface-coated with an insulin-chitosan complex.
Fig. 2 shows the results of formation of an insulin-chitosan complex on the surface of PLGA small particles, wherein the left histogram shows release of insulin in a buffer having pH 6.0, the right histogram shows release of insulin in a buffer having pH 4.5, and the vertical axis shows the amount ($\mu$g) of released insulin.
Fig. 3 shows results from Time of Flight Secondary Ion Mass Spectrometry (ToF-SIMS) analysis of PLGA core particles surface-coated with an insulin-chitosan complex, wherein the upper panel shows detection results of the m/z=33 peak in each sample (PLGA, chitosan (Chitosan), insulin (Insulin) and surface-coated small particles (Carrier System)) (horizontal axis: m/z; vertical axis: detection intensity), and the lower panel shows a distribution image (left) of the m/z=33 peak of surface-coated small particles and a total ion distribution image (right) in the observation field.
Fig. 4 shows the proportion (%) of non-degraded insulin left in the chymotrypsin solution after culture, where the upper bar shows the free insulin solution and the lower bar corresponds to PLGA/chitosan/insulin surface-coated small particles.
Fig. 5 is a schematic diagram that explains the method used for the insulin transport study through a porcine nasal mucous membrane.
Fig. 6 shows the results of an insulin transport study for porcine nasal mucous membrane, wherein the horizontal axis shows the time (min) after the start of the transport study, and the vertical axis shows the insulin amount (ng/cm$^2$) that was transported across the porcine nasal mucous membrane in a given time.
Fig. 7 shows the results of particle size measurement of surface-coated small particles or chitosan/insulin mixture, wherein (a) shows particle size distribution of surface-coated small particles (having core particles; PLGA100/chitosan/insulin=1250/900/200 $\mu$g/ml), (b) shows particle size distribution of a chitosan/insulin mixture (free of core particles; chitosan/insulin=900/200 $\mu$g/ml), the horizontal axis shows particles radius (nm) and the vertical axis shows intensity.
Fig. 8 shows the results of particle size measurement of surface-coated small particles (PLGA100/chitosan/insulin=250/180/40 $\mu$g/ml) before freeze-drying and after freeze-drying and resuspending wherein (a) shows particle size distribution of surface-coated small particles before freeze-drying and (b) shows particle size distribution of surface-coated small particles after freeze-drying and resuspending, the horizontal axis shows particles radius (nm) and the vertical axis shows intensity.

Fig. 9 shows blood glucose level after nasal administration of the sample from Example 7 or a control sample to rats, wherein the results show a relative decrease in the glucose level (%) to that before administration of the sample as 100%, mean value $\pm$ standard error.

Fig. 10 shows the results of an in vitro release test using the sample from Example 8 (N=3) (5 mM MES isotonic buffer (pH 6), 37°C).

**Description of Embodiments**

The present invention provides a pharmaceutical composition for transmucosal administration, comprising (a) a drug having a positive or negative charge at a predetermined pH, (b) a pharmaceutically acceptable small particle and (c) a pharmaceutically acceptable surface-coating polymer capable of being electrically charged at the pH. In the composition, the surface of the small particle is coated by the surface-coating polymer, the drug is immobilized on the surface of the small particle via the surface-coating polymer, and a complex (hereinafter to be also referred to as a "surface-coated small particle") is formed by a noncovalent interaction between the small particle and the surface-coating polymer and a concurrent electrostatic interaction between the surface-coating polymer and the drug.

The "pharmaceutical composition for transmucosal administration" means a pharmaceutical composition that is administered to a mucosa of a subject in need of a treatment and/or a therapy by an appropriate method such as coating, spraying, nebulizing, applying and the like, wherein the drug can be delivered to a mucosal tissue or to the circulatory system or immune system across a mucosal tissue to produce a medicinal or vaccine effect. The pharmaceutical composition for transmucosal administration can be, for example, absorbed across the mucosa for systemic delivery. As examples of mucosa, mucosas such as those in lung, mouth cavity, eye, vagina, gastrointestinal tract, nose and the like can be mentioned. From the point of view of convenience of administration, nasal mucosa is preferable as the mucosa.

The pharmaceutically acceptable surface-coating polymer of the above-mentioned (c), which constitutes the surface-coated small particle, covers the surface of small particles as mentioned above. The "coating" here simply means that the surface-coating polymer is attached to the surface, rather than the inside, of the small particle by a noncovalent interaction between the small particle and the surface-coating polymer, and does not necessarily mean that the entire surface of the small particle is coated with the surface-coating polymer.

The surface-coating polymer is preferably biocompatible. The term "biocompatibility" herein means that a substance and degradation products thereof have no toxic or hazardous effect on a body tissue or a body system (e.g., blood circulation system, nerve system, immunity system and the like). The biocompatible polymer is suitable for administration to human or other animals. In addition, the surface-coating polymer is more preferably biodegradable. The term "biodegradability" herein means that a substance is degraded within a living body by an enzymatic, chemical or physical process or the like within an acceptable period of time to form smaller chemical species. Methods for examining biocompatibility and biodegradability of a substance are well known in the technical field of the invention.

The surface-coating polymer may be a natural polymer or a synthetic polymer. Since the surface-coating polymer ionically bonds with the drug on the surface of the small particle at the predetermined pH, it needs to be a polymer having a charge of the sign opposite to that of the drug at the predetermined pH. Where necessary, more than one surface-coating polymer can be used in combination for the surface-coating polymer as long as they are capable of having charges of the same sign at the predetermined pH.

The pharmaceutical composition of the present invention can be produced, for example, by the below-mentioned production method; when the composition is produced by the production method, even if the surface-coating polymer is by itself slightly water-soluble at the predetermined pH, the composition can be produced by mixing the surface-coating polymer with the drug and the small particles at a pH where the surface-coating polymer is readily water-soluble, and then adjusting the pH of the mixture to the predetermined pH. Therefore, for the surface-coating polymer, not only polymers that are readily water-soluble at the predetermined pH, but also polymers that are slightly water-soluble at the predetermined pH can be used.

Polymers that can be used for the surface-coating polymer, can be selected from but are not limited to polyanionic or polycationic polysaccharides, polyamino acids and other charged polymers. The polymer is appropriately selected based on the type of the drug used, the charge of the surface-coating polymer and of the drug and the like.

Polyanionic polysaccharides that can be used in the present invention means a polysaccharide that has one or more acidic polar groups such as a carboxyl group, a sulfuric acid group or a phosphoric acid group in the constitutional unit. Examples of such polyanionic polysaccharides include, but are not limited to, chondroitin sulfuric acid, dextran sulfuric acid, carboxymethylcellulose, alginic acid, pectin, hyaluronic acid, derivatives and salts thereof and the like.

Polycationic polysaccharides that can be used in the present invention means a polysaccharide that has one or more basic polar group such as an amino group in the constitutional unit. Examples of such polycationic polysaccharides include, but are not limited to, chitin, chitosan, derivatives and salts thereof and the like. The chitosan and the chitosan derivatives can be selected from those having a various molecular weights, degrees of deacetylation and, for the chitosan derivatives, degrees of substitution.

The polyanionic polyamino acid that can be used in the present invention means a polyamino acid whose isoelectric point is on the acidic side of the physiological pH; examples thereof include, but are not limited to, polyglutamic acid, polyaspartic acid, derivatives and salts thereof and the like.

The polycationic polyamino acid that can be used in the present invention means a polyamino acid whose isoelectric point is on the basic side of the physiological pH; examples thereof include, but are not limited to, polylysine, polyarginine, derivatives and salts thereof and the like.

Examples of the polymer that can be used for the surface-coating polymer other than the above-mentioned polysaccharides and polyamino acids, include polyethylenimine, polyacrylic acid, derivatives and salts thereof and the like.

The surface-coating polymer may be polyethylene glycolated (PEGylated) and/or glycosylated.

The surface-coating polymer may further be mucoadhesive and/or act as a transmucosal absorption promoter. Examples of mucoadhesive polymers include chitosan, polyacrylic acid, sodium alginate, carboxymethylcellulose and the like as well as PEGylated polymers thereof and the like. Examples of the polymer that acts as a transmucosal absorption promoter include chitosan, polyacrylic acid, polyarginine, salts and derivatives thereof and the like.

Those ordinarily skilled in the art can determine the molecular weight of a surface-coating polymer in consideration of factors such as degradation rate, mechanical strength, solubility, which kind of drug would form the complex with the surface-coating polymer, and the like. Typically, the weight average molecular weight of the surface-coating polymer should preferably be not less than 1,000 Da, and more preferably not less than 2,000 Da; preferably not more than 1,000,000 Da, and more preferably not more than 500,000 Da, as measured by gel permeation chromatography. Accordingly, typically, the weight average molecular weight of the surface-coating polymer is preferably between 1,000-1,000,000 Da, and more preferably between 2,000-500,000Da. For example, the weight average molecular weight of chitin or chitosan may be between 1,000-1,000,000 Da. and the degree of deacetylation of chitin or chitosan may be between 20-100%.

The composition of the present invention enables control of the rate of release of the drug as a sustained-release or immediate-release composition and the regulation of transmucosal absorbability of the drug based on the selection of the surface-coating polymer. Those ordinarily skilled in the art can appropriately select the surface-coating polymer so as to afford the desired pharmacokinetic property of the composition.

The drug used for the composition of the present invention is selected according to the intended use. In the composition of the present invention, since the drug ionically binds with an appropriate surface-coating polymer on the surface of the small particle, a drug that can be used in this composition needs to be charged positively or negatively (i.e., having a charge of the sign opposite to that of the surface-coating polymer) at the predetermined pH. As long as this condition is met, any drug can be used for the composition of the present invention. Where necessary, more than one drug can be used in combination as long as they are capable of having charges of the same sign at the predetermined pH.

The drug can be, without limitation, a peptide, a protein, a DNA, an RNA, an siRNA, a polysaccharide, an antibody, an antigen, a low-molecular weight compound and the like. The pharmaceutical composition of the present invention can be produced, for example, by the below-mentioned production method. When the composition is produced by the below-mentioned production method, a drug, permitting variation of charge (sign and intensity) based on a pH change during the preparation process, can be particularly preferable, because it is possible to sufficiently mix the drug and the surface-coating polymer under charge conditions free of an electrostatic interaction between the drug and the surface-coating polymer, and then form an ion bond between them by changing the pH. Examples of such drugs include amphoteric drugs such as peptides and proteins, which can be positively or negatively charged depending on the pH, drugs having such acid dissociation constant (pKa) or base dissociation constant (pKb) as changes the charge intensity markedly between in the preparation process and in the composition, and low-molecular weight drugs in the form of salts such as hydrochloride, sulfate, acetate and the like, which are capable of dissolving in water and having a charge that is less dependent on pH.

Examples of drugs include, but are not limited to, antihypertensive agent, antihypotensive agent, analgesic, antipsychotic agent, antidepressant, antimanic, antianxiety agent, sedative, hypnotic, antiepileptic, opioid agonist, therapeutic agent for asthma, anesthetic, antiarrhythmic agent, therapeutic agent for arthritis, anticonvulsant, ACE inhibitor, decongestant, antibiotic, antianginal agent, diuretic, antiparkinson agent, bronchodilator, oxytocic, antidiuretic, antilipemic agent, immunosuppressant, immunity regulator, antiemetic, antiinfective agent, antineoplastic, antifungal agent, antivirus agent, antidiabetic agent, antiallergic agent, fever reducer, antitumor agent, antigout agent, antihistamine agent, antipruritic agent, bone regulator, cardiovascular agent, hypocholesterolemic agent, antimalarial agent, pharmaceutical agent for ceasing smoking, antitussive agent, expectorant, mucolytic agent, nasal decongestant, dopamine agonist, pharmaceutical agent for digestive tract, muscle relaxant, neuromuscular blocker, parasympatholytic, prostaglandin, stimulant drug, anorectic agent, thyroid agent or antithyroid agent, hormone, antimigraine agent, antiobesitic agent, antiinflammatory agent and the like. The drug may also be selected from a variety of peptides, proteins, polysaccharides, antigens, antibodies, DNAs, RNAs, siRNAs, low-molecular weight drugs and the like, which are intended for prophylactic vaccination, immunotherapy, antibody therapy, gene therapy, suppression of gene expression and the like.

Specific examples of the drug include, but are not limited to, insulin, glucagon, leuprolide, growth hormones, parathyroid hormones, calcitonin, vascular endothelial growth factor, erythropoietin, heparin, cyclosporin, oxytocin, tyrosine, enkephalin, tyrotropin releasing hormone, follicle-stimulating hormone, leuteinising hormone, vasopressin, vasopressin analogs, catalase, superoxide dismutase, interleukin II, interferons, colony stimulating factor, tumor necrosis factor, melanocyte stimulating hormone, glucagon-like peptide-1, glucagon-like peptide-2, katacalcin, cholecystekinin-12, cholecystekinin-8, exendin, gonadoliberin-related peptide, insulin-like protein, leucine-enkephalin, methionine-enkephalin, leumorphine, neurophysin, copeptin, neuropeptide Y, neuropeptide AF, PACAP-related peptide, pancreatic hormone, peptide YY, urotensin, intestinal peptide, adrenocorticotropic peptide, epidermal growth factor, prolactin, luteinising hormone releasing hormone (LHRH), LHRH agonist, growth hormone releasing factor, somatostatin, gastrin, tetragastrin, pentagastrin, endorphins, angiotensins, tyrotropin releasing hormone, granulocyte-colony stimulating factor, granulocyte-macrophage-colony stimulating factor, heparinase, antigens for influenza vaccine, tetanus toxins, peptides for cancer vaccine, β-amyloid, immunoglobulins, siRNAs for treatment of cirrhosis, siRNAs for treatment of cancer, low-molecular weight drugs such as bromhexine, granisetron, zolmitriptan, sumatriptan and the like, and pharmaceutically acceptable salts thereof, and the like.

As mentioned above, the drug and the surface-coating polymer bind to each other by an electrostatic interaction on the surface of the small particle to form a drug-surface-coating polymer complex. Regarding the combination of the drug and the surface-coating polymer forming the complex, it may be a combination where the drug is positively charged and the surface-coating polymer is negatively charged at the predetermined pH, or a combination where the drug is negatively charged and the surface-coating polymer is positively charged at the predetermined pH.

The above-mentioned pharmaceutically acceptable small particle (while the term "small particle" in the present specification means the above-mentioned small particle (b), the small particle may sometimes be referred to as a "small core particle" so as to clearly distinguish this term from a "surface-coated small particle") is preferably composed of biocompatible polymer(s). The polymer may be biodegradable or non-biodegradable; from the viewpoint of safety to a living body, a biodegradable one is preferable. The polymer may be a natural polymer or a synthetic polymer.

Examples of the biocompatible and biodegradable polymer that can be used for the small particle include, but are not limited to, polyethylene glycol (PEG), polylactic acid (PLA), poly(glycolic acid) (PGA), poly(lactic acid-glycol acid) copolymer (PLGA), block copolymers of PEG and PLGA (PEG-PLGA), polyanhydrides, poly(ε-caprolactone), polyhydroxybutyrate, polyamino acids, polyortho esters, polyphospho esters, polydiaxanone, polyester amides, polyphosphagen, polycyano acrylate, chitosan, chitosan derivatives, starch, starch derivatives, albumin, fibrin, fibrinogen, cellulose, collagen, hyaluronic acid, mixtures and copolymers of these substances, and the like.

Examples of the biocompatible and non-biodegradable polymer that can be used for the small particle include, but are not limited to, polyacrylate, polyacrylate esters, poloxamer, tetronics, polyethylene, polymethyl methacrylate, polymethyl methacrylate esters, polystyrene, ethylene vinyl acetate, acylated cellulose acetate, polyurethane, polyvinyl chloride, mixtures and copolymers of these substances, and the like.

The small particle may be hydrophilic or hydrophobic. Since the shape and size of the small particle can be easily maintained in the preparation process of the small particle and the preparation process of the surface-coated small particle in a water system, a hydrophobic small particle is preferred. As preferable examples, hydrophobic polymers having a carboxyl group, or a primary, secondary or tertiary amino group can be used for the small particle.

Those ordinarily skilled in the art can determine the molecular weight of the polymer for the small particle by considering the factors such as degradation rate, mechanical strength and solubility. Typically, the weight average molecular weight of the polymer as measured by gel permeation chromatography is preferably not less than 1,000 Da (Dalton), and more preferably not less than 2,000 Da; preferably not more than 1,000,000 Da, and more preferably not more than 500,000 Da. Accordingly, typically, the weight average molecular weight of the polymer is preferably between 1,000-1,000,000 Da, and more preferably between 2,000-500,000Da.

Regarding the size of the small core particle, a particle having an average particle size of not less than 1 nm, preferably not less than 5 nm, and more preferably not less than 10 nm, and one having an average particle size of not more than 50 μm, preferably not more than 20 μm, and more preferably not more than 10 μm, can be mentioned. The particle size here refers to a value obtained by measuring small particles dispersed in an aqueous solution at the aforementioned "predetermined pH". The particle size is a diameter measured by a particle size measuring apparatus and calculated on the assumption that the particles have a spherical shape. The particle size measuring apparatus and the calculation method of the average particle size are appropriately changed according to the particle size. To be specific, in the case of a particle size measurable by a dynamic light scattering measuring apparatus (generally not more than 7 μm), the size is measured by a dynamic light scattering measuring apparatus, and an average of the hydrodynamic diameter determined from the scattering intensity distribution is employed as an average particle size. In the case of a large particle size immeasurable by a dynamic light scattering measuring apparatus (generally greater than 7 $\mu$m), the size is measured by a laser diffraction system particle size distribution measuring apparatus, and an average diameter obtained by arithmetically averaging the frequency distribution is employed as an average particle size.

Here, an average particle size of small core particles of, for example, not less than 10 nm means that not less than 10%, preferably not less than 20%, more preferably not less than 30%, still more preferably not less than 40%, particularly preferably not less than 50% of the average particle size of a particle size peak, is not less than 10 nm in the proportion of each particle size peak in the above-mentioned scattering intensity distribution by the dynamic light scattering measuring apparatus (proportion of cumulative scattering intensity for each particle size peak to cumulative scattering intensity for all peaks), or the proportion of each particle size peak in the above-mentioned frequency distribution by the laser diffraction system particle size distribution measuring apparatus (proportion of cumulative frequency for each particle size peak to cumulative frequency for all peaks).

When the composition of the present invention is administered to the mucosa, the surface-coated small particle reaches the mucosal surface or may get taken up into the mucosal tissue and releases the drugs there. Then the drug is transported into the bloodstream. When the small particle is sufficiently small (e.g., particle size of the small particle of not more than 20 nm), it may pass through the intercellular gap to reach the bloodstream. Alternatively, the small particle may be ingested by a M-cell or M-like cell in some mucosa such as the nasal or the intestinal and transported into the immune system or lymphatic system.

The above-mentioned small particle can be produced by various methods described in the literature. Examples of the literature include Champion JA. et al., Proc. Natl. Acad. Sci. USA, Vol.104, pp.11901-4 (2007); Chattopadhyay P. et al., Adv. Drug Deliv. Rev., Vol.59, pp.443-53 (2007); Zhou WY et al., J. Mater. Sci. Mater. Med., Vol.19, pp.103-110 (2008); Schaffazick SR et al., Pharmazie, Vol.62, pp.354-60 (2007); Almeida AJ et al., Adv. Drug Deliv. Rev., Vol.59, pp.478-90 (2007); Muller, R.H., "Colloidal Carriers for Controlled Drug Delivery and Targeting: Modification, Characterization and In vivo Distribution", CRC Press (1991); Jorg Kreuter (ed.), Colloidal Drug Delivery Systems, Marcel Dekker (1994) and the like. Examples of the methods that can be used for the production of the small particle include nanoprecipitation, phase separation, emulsion, self assembly, high-pressure homogenization, complexation, ionic gelation and the like.

As mentioned above, the small particle needs to noncovalently interact with the surface-coating polymer to create the surface-coated small particle. Here, the noncovalent interaction means interactions not based on covalent bond, such as electrostatic interaction, hydrophobic interaction, van der Waals interaction, hydrogen bonding and the like. Among them, for example, when electrostatic interaction is utilized, the small particle needs to be a polymer having a charge of the sign opposite to that of the surface-coating polymer at a predetermined pH in order to allow electrostatic interaction. Accordingly, the combination of the drug, the surface-coating polymer and the polymer for the small particle, which are to be used for the composition, is a combination of a positively-charged drug, a negatively-charged surface-coating polymer, and a positively-charged polymer for the small particles, all of which at a predetermined pH, or a combination of a negatively-charged drug, a positively-charged surface-coating polymer, and a negatively-charged polymer for the small particles, all of which at a predetermined pH. Requirements of the isoelectric point (pI), acid dissociation constant (pKa) and base dissociation constant (pKb) that are to be satisfied by the drug, the surface-coating polymer and the polymer for small particles so as to achieve such combination are as follows: the value of pI or pKa or (14-pKb) of the drug and the polymer for small particles is higher than the pH of the composition after production, and the value of pI or pKa or (14-pKb) of the surface-coating polymer is lower than the pH of the composition after production; or the value of pI or pKa or (14-pKb) of the drug and the polymer for small particles is lower than the pH of the composition after production, and the value of pI or pKa or (14-pKb) of the surface-coating polymer is higher than the pH of the composition after production. Determination of the isoelectric point and/or acid dissociation constant for each compound is within the general technical scope of those ordinarily skilled in the art. Alternatively, in case of low-molecular weight drugs in the form of salts such as hydrochloride, sulfate, acetate and the like, which are capable of dissolving in water and having a charge, the composition can be prepared by combining a charged drug with a surface-coating polymer having a charge of the sign opposite to that of the drug in water and a small particle having a charge of the sign same as that of the drug in water.

The predetermined pH, i.e., the pH of the composition after production, is desirably set to the physiological pH of the administration site to avoid topical irritation. As mentioned above, the composition of the present invention can be administered to mucosa such as that in the lung, mouth cavity, eye, vagina, intestine, nose and the like, where the physiological pH varies in these various mucosas. For example, the physiological pH of the gastrointestinal tract increases along the length thereof from about pH 1 in the stomach to pH 8 in the colon; the mouth cavity has a pH around 6.8; the pH of nasal fluid is within the range of about pH 5.5 to 6.5; the pH of vagina is around 4.5. For example, when the composition of the present invention is to be administered to the nasal mucosa, preferable pH value of the composition is, for example, about 6.0.

As mentioned above, insulin can be used as the drug in the composition of the present invention. When the pH of the composition is 6.0, insulin is negatively charged in the composition since the isoelectric point of insulin is about pH 5.3. Hence, the surface-coating polymer has to be a polymer having a positive charge at pH 6.0. When electrostatic interaction is utilized as the noncovalent interaction to make the surface-coating polymer and the small particle interact with each other, the small particle composed of a polymer having a negative charge at pH 6.0 can be used as a preferable small particle. Such surface-coating polymer may be chitosan, and the polymer for the small particle may be a poly(lactic acid-glycol acid) copolymer (PLGA).

As mentioned above, bromhexine, zolmitriptan and salts thereof can be used as the drug in the composition of the present invention. When the pH of the composition is 6.0 to 7.0, the surface-coating polymer has to be a polymer having a negatively charge at said pH, since the drug is positively charged in water. When electrostatic interaction is utilized as the noncovalent interaction to make the surface-coating polymer and the small particle interact with each other, a small particle composed of a polymer having a positive charge at said pH can be used as a preferable small particle. Examples of such surface-coating polymer include polyacrylic acid, poly-gamma-glutamic acid and salts thereof, and examples of the small particle include chitosan small particle and amino-modified polystyrene particle.

Regarding the size of the surface-coated small particles, a particle having an average particle size of not less than 10 nm, preferably not less than 20 nm, more preferably not less than 40 nm, and one having an average particle size of not more than 50 $\mu$m, preferably 20 $\mu$m, more preferably not more than 10 $\mu$m can be mentioned.

The particle size here refers to a value obtained by measuring surface-coated small particles dispersed in an aqueous solution at the aforementioned "predetermined pH". To be specific, when the surface-coated small particles are in the form of a suspension, the size is measured after diluting the suspension to a concentration suitable for the measurement with an aqueous solution having the same pH (the aforementioned predetermined pH) as the pH of the suspension. When the surface-coated small particles are in the dosage form other than suspension, such as dry powder, sheet and the like, which prevents direct measurement of the particle size, water or a suitable pH buffer is added to prepare a suspension having the aforementioned "predetermined pH" and then the particle size is measured. The particle size is a diameter measured by a particle size measuring apparatus and calculated on the assumption that the particles have a spherical shape. The particle size measuring apparatus and the calculation method of the average particle size are appropriately changed according to the particle size. To be specific, in the case of a particle size measurable by a dynamic light scattering measuring apparatus (generally not more than 7 $\mu$m), the size is measured by a dynamic light scattering measuring apparatus, and an average of the hydrodynamic diameter determined from the scattering intensity distribution is employed as an average particle size. In the case of a large particle size immeasurable by a dynamic light scattering measuring apparatus (generally greater than 7 $\mu$m), the size is measured by a laser diffraction system particle size distribution measuring apparatus, and an average diameter obtained by arithmetically averaging the frequency distribution is employed as an average particle size.

Here, an average particle size of surface-coated small particles of, for example, not less than 10 nm means that not less than 10%, preferably not less than 20%, more preferably not less than 30%, still more preferably not less than 40%, particularly preferably not less than 50% of the average particle size of a particle size peak, is not less than 10 nm in the proportion of each particle size peak in the above-mentioned scattering intensity distribution by the dynamic light scattering measuring apparatus (proportion of cumulative scattering intensity for each particle size peak to cumulative scattering intensity for all peaks), or the proportion of each particle size peak in the above-mentioned frequency distribution by the laser diffraction system particle size distribution measuring apparatus (proportion of cumulative frequency for each particle size peak to cumulative frequency for all peaks).

Owing to the presence of the small particle as the core, the surface-coated small particle in the composition of the present invention has a monodispersed particle size compared to a complex formed by simply mixing a surface-coating polymer and a drug. Accordingly, the constitution of the surface-coated small particle as in the present invention makers it easy to prepare a preparation with a uniform property. This characteristic is also an advantage of the present invention.

The composition of the present invention needs to be delivered as a preparation permitting the surface-coated small particle to directly reach the target mucosal site. Examples thereof include pulmonary agent, oral agent, buccal agent, intraocular agent, vaginal agent, intranasal agent, suppository and the like.

As the pulmonary agent, an inhalant which is delivered to alveoli by a pulmonary inhaler device is preferred.

As the oral agent, usual oral preparations, for example, tablet, granule, fine granule, capsule and the like can be mentioned. Dosage forms designed to release the drug in the small intestine, for example, enteric coated tablet, enteric coated granule, enteric coated capsule and enteric coated fine granule are preferred.

As the buccal agent, the intraocular agent and the intranasal agent, buccal tablet, buccal spray, eye drop, nose drop, aerosol, ointment, gel, cream, liquid, suspension, lotion, dry powder, sheet, patch and the like can be mentioned.

As the vaginal agent and suppository, ointment, gel, cream, liquid, suspension, lotion, dry powder, sheet, capsule and the like can be mentioned.

As methods for preparing the above-mentioned dosage forms, known production methods generally used in the field can be applied. Where necessary, when preparing the above-mentioned dosage forms, carriers such as excipient, binder, disintegrant and lubricant, and various preparation additives such as sweetening agents, surfactants, suspending agents, emulsifiers, colorants, preservatives and stabilizers, which are generally used for preparing the particular dosage form can be appropriately added in an appropriate quantity to produce the dosage forms. Also, the composition of the present invention can be preserved in the form of a dry powder prepared by lyophilizing the suspension, and the like, and resuspended by adding water to the dry powder when in use. Employing this method, hydrolysis of the drug, the polymer for the small particle and/or the surface-coating polymer can be avoided in order to improve the preservation stability of the composition.

The preferable relative proportions of the polymer for the small particle, the surface-coating polymer and the drug in the composition of the present invention vary depending on the small particle, the surface-coating polymer and the drug to be used and hence cannot be stated in general. For example, when a poly(lactic acid-glycol acid) copolymer (PLGA) is used as the polymer for the small particle, chitosan is used as the surface-coating polymer, and insulin is used as the drug, the weight ratio thereof in the composition can be PLGA:chitosan:insulin=1:0.1-100:0.01-100.

The pharmaceutical composition of the present invention is stable and of low toxicity, and can be used safely. The administration frequency and single dose vary dependent on the drug used, condition and body weight of patient, administration route, therapeutic strategy and the like and hence cannot be stated in general. For example, when the composition of the present invention in which insulin is used as the drug, is transnasally administered to a patient with diabetes and the like, as one therapeutic strategy, about 2 mg to about 6 mg of the active ingredient (insulin) can be administered to an adult (about 60 kg in body weight) before each meal.

The present invention also provides a production method of the aforementioned pharmaceutical composition. The method of the present invention comprises mixing the drug, the surface-coating polymer and the small particles in a solution with a suitable pH, optionally changing the pH to induce an electrostatic interaction between the drug and the surface-coating polymer and a noncovalent interaction between the surface-coating polymer and the small particle. The method needs no heating treatment and the like and therefore is convenient.

In the method, the combination of the drug, the surface-coating polymer and the small particles and the pH of the composition of the present invention (the predetermined pH) are determined in advance. These factors can be determined as mentioned above in the explanation on the composition of the present invention. The small particles are generally prepared by the aforementioned method prior to mixing the drug, the surface-coating polymer and the small particles. Then, the drug, the surface-coating polymer and the small particles are mixed, and optionally the pH is adjusted, whereby the surface-coated small particle of the present invention is produced. The mixture and the optional pH adjustment comprise any one selected from the group consisting of the following a) to c):

a) mixing the drug and the surface-coating polymer in a solution with a pH at which the complex thereof is not formed, adding the small particle into the solution, and changing the pH of the solution to promote formation of the complex of the drug and the surface-coating polymer and immobilization of the drug-surface-coating polymer complex on the surface of the small particle;

b) mixing the drug and the surface-coating polymer in a solution with a pH at which the complex thereof is formed, and adding the small particles into the solution to make the drug-surface-coating polymer complex immobilized on the surface of the small particles;

c) mixing the small particle and the surface-coating polymer in a solution to make the surface-coating polymer immobilized on the surface of the small particle, adding the drug into the solution, and adjusting the pH of the solution to promote formation of the complex of the drug and the surface-coating polymer immobilized on the surface of the small particle.

Particularly, when the surface-coating polymer is by itself slightly water-soluble at the predetermined pH, it is desirable first (a) to mix the drug, the small particle and the surface-coating polymer at a pH at which the surface-coating polymer is readily water-soluble, then (b) to adjust the pH of the mixture to the predetermined pH.

When a drug that changes the sign of the charge depending on pH, i.e., an amphoteric drug, is used as the drug for the pharmaceutical composition of the present invention, the following method can be utilized for the production method of the pharmaceutical composition of the present invention. That is, as the first step, the drug, the surface-coating polymer and the small particle are mixed under a pH condition under which the charge of the drug and the charge of the surface-coating polymer are of the same sign, thereby allowing both the drug and the surface-coating polymer to be drawn toward the surface of the small particle due to a noncovalent interaction such as an electrostatic interaction. Additionally, as the second step, the pH of the mixture is adjusted to a pH at which the charge of the drug changes to the opposite sign, thus efficiently forming a bond between the drug and the surface-coating polymer assembled on the surface of the small particle by an electrostatic interaction, whereby the pharmaceutical composition of the present invention can be produced efficiently. This production method is useful since it can suppress generation of a free drug-surface-coating polymer complex (not immobilized on the surface of the small particle) as a by-product.

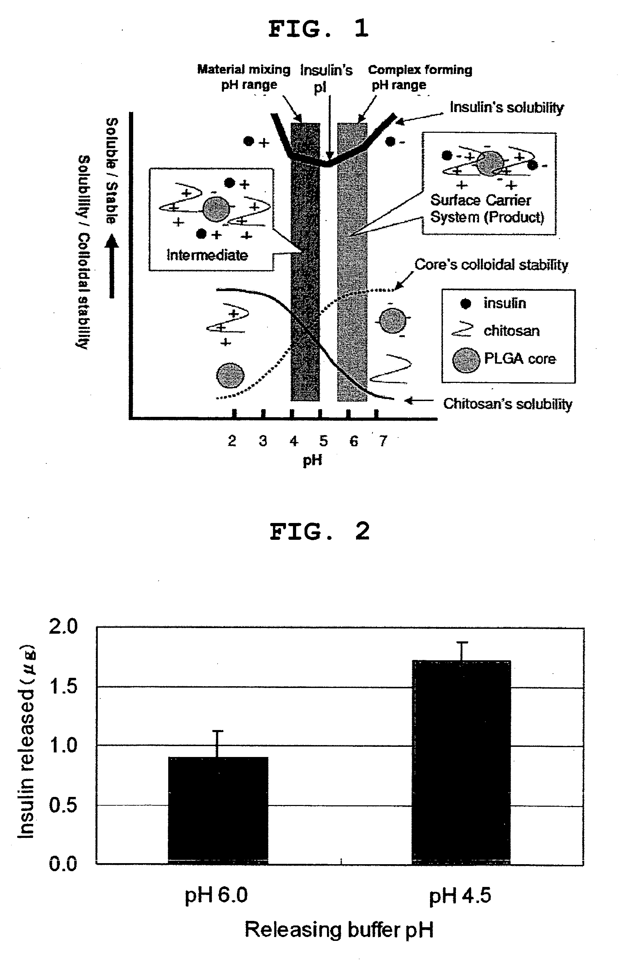

Explaining this method with insulin (drug; isoelectric point: about 5.3), chitosan (surface-coating polymer) and PLGA small particle (small particle) for example, insulin, chitosan and PLGA small particle are mixed at "a pH less than the isoelectric point" where insulin is positively charged (e.g., pH 4.5 and the like), then the pH is adjusted to "a pH higher than the isoelectric point" at which insulin is negatively charged (pH 6.0 and the like). While chitosan has a positive charge and PLGA particle has a negative charge at both pH 4.5 and pH 6.0, insulin is positively charged at pH 4.5 and negatively charged at pH 6.0. Hence, one of the compositions of the present invention can be produced wherein mutually ionically-bonded chitosan and insulin are immobilized on the surface of the PLGA small particle at pH 6.0. Fig. 1 explains this embodiment.

Accordingly, the present invention provides a production method of the composition of the present invention, comprising

> (a) mixing the drug, the surface-coating polymer and the small particle under a pH condition under which the drug and the surface-coating polymer have the same sign of the charge, and then
> (b) adjusting the pH of the mixture to a pH at which the sign of the charge of the drug changes to the opposite sign, wherein the drug is an amphoteric drug.

Alternatively, as a still another production method especially useful for minimizing the particle size of the surface-coated small particle as a product by reducing the particle size of the core particle, the following method can be utilized. In this method, preparation of the small particle and an electrostatic interaction between the small particle and the surface-coating polymer are simultaneously started, rather than preparing the small particle in advance. Specifically, in this method, a suitable organic solvent (e.g., acetone solution and the like) containing the material of the small particle as mentioned above is added dropwise into an aqueous solution of the surface-coating polymer, and the organic solvent is evaporated from the solution by agitation and the like, whereby formation of small particle as the core particle and coating of the small particle with the surface-coating polymer are started; after which the drug is added and mixed, the pH is optionally changed to promote an electrostatic interaction between the drug and the surface-coating polymer and between the surface-coating polymer and the small particle, whereby the surface-coated small particle is produced.

Accordingly, the present invention provides a production method of the composition of the present invention, comprising

> (a) adding dropwise an organic solvent solution of a material of the small particle into an aqueous solution of the surface-coating polymer,
> (b) evaporating the organic solvent,
> (c) adding the drug and stirring the mixture, and
> (d) adjusting the pH of the mixture to the predetermined pH.

While the present invention is hereinafter further explained in detail by referring to Examples and Experimental Examples, the present invention is not limited by the following Examples and the like.

**Examples.**

Preparation Example 1: Preparation of poly(lactic-co-glycolic acid) (PLGA) small particles of various particle sizes

PLGA small particles were produced using a PLGA with a lactide:glycolide ratio of 50:50 (RESOMER RG 502H, Bohringer Ingelheim). The PLGA was dissolved in HPLC grade acetone at the required concentration. The PLGA/acetone solution was added dropwise to purified water in a ratio of 1:3 under constant stirring. The mixture was stirred until the acetone had fully evaporated (approximately 4 hours).

The particle size distribution of resultant small particles was measured by a dynamic light scattering measuring apparatus (DLS 802, Viscotek). Table 1 shows the relationship between the PLGA concentration and the diameter of obtained particle. It is evident that by reducing the polymer concentration in the initial organic solvent solution, smaller particles can be easily and reproducibly obtained.

Table 1
Effect of PLGA concentration on particle size

| LGA concentration in acetone (% (w/v)) | PLGA small particle diameter (nm) | |
|---|---|---|
| | mean | standard deviation |
| 3.00 | 177 | 50 |
| 1.00 | 111 | 27 |
| 0.30 | 78 | 26 |
| 0.10 | 46 | 11 |

(continued)

Effect of PLGA concentration on particle size

| LGA concentration in acetone (% (w/v)) | PLGA small particle diameter (nm) | |
|---|---|---|
| | mean | standard deviation |
| 0.05 | 36 | 15 |
| 0.01 | 20 | 6 |
| 0.005 | 16 | 4 |

Example 1: Preparation of small particle system surface-coated with drug-surface-coating polymer complex in two different buffer systems

Insulin (pI about 5.3) was used as a protein drug, and chitosan was used as a positively charged surface-coating polymer.

1.5 ml of bovine insulin (Sigma, 160 $\mu$g/ml) in 0.5 mM citric acid solution (pH 4.5) was added to 1.5 ml of chitosan (Bioneer 143 kDa, 0.72 mg/ml) in 0.5 mM citric acid solution (pH 4.5) and the mixture was left at room temperature for at least 30 min. Three ml of PLGA small particles (about 100 nm in diameter; hereinafter, also to be referred as "PLGA 100") suspension in 0.5 mM citric acid solution (pH 4.5; concentration of PLGA small particle: 500 $\mu$g/ml) prepared as described in Preparation Example 1 was added to the chitosan/insulin solution and the mixture was left at room temperature for at least 1 hour. The pH was increased to 6.0 with NaOH (0.1-2.5N), and salts and supplements were added thereto to afford the same solvent compositions of the suspension as that of the buffers described in Table 2 or Table 3.

Table 2

Composition of 0.5 mM citric acid isotonic buffer (pH 6.0)

| | MW | g/L | concentration (mM) | ion intensity (mM) |
|---|---|---|---|---|
| D-glucose | 180.16 | 1.80 | 10 | ---- |
| MgCl$_2$ | 95.21 | 0.0468 | 0.492 | 1.476 |
| KCl | 74.55 | 0.340 | 4.56 | 4.56 |
| CaCl$_2$·2H$_2$O | 147.02 | 0.176 | 1.2 | 3.6 |
| citric acid | 192.1 | 0.096 | 0.5 | 0.8 |
| NaCl | 58.44 | 8.015 | 137.15 | 137.15 |

Table 3

Composition of 50 mM MES 0.5 mM citric acid isotonic buffer (pH 6.0)

| | MW | g/L | concentration (mM) | ion intensity (mM) |
|---|---|---|---|---|
| D-glucose | 180.16 | 1.80 | 10 | ---- |
| MgCl$_2$ | 95.21 | 0.0468 | 0.492 | 1.476 |
| KCl | 74.55 | 0.340 | 4.56 | 4.56 |
| CaCl$_2$·2H$_2$O | 147.02 | 0.176 | 1.2 | 3.6 |
| citric acid | 192.1 | 0.096 | 0.5 | 0.8 |
| MES | 195.2 | 9.76 | 50 | 20 |
| NaCl | 58.44 | 7.22 | 123.56 | 123.56 |

The particle size and the zeta potential of the surface-coated small particles were measured by DLS 802 (Viscotek) and Zeta sizer 2000 (Malvern), respectively (Table 4).

As can be seen from Table 4, similar and preferable particle sizes and zeta potentials were obtained for the two different buffer systems. The two kinds of surface-coated small particles each had a particle size about two-fold of that of the uncoated small particles, and a highly positive zeta potential. Both of the surface-coated small particles were found to be stable as colloidal suspensions.
Table 4

Surface-coated PLGA small particles in two different buffer systems (PLGA 100/CS/Ins)

|  | diameter (nm) | zeta potential (mV) |
| --- | --- | --- |
| uncoated PLGA small particle | 125.6 ± 33.0* | - 64.6 ± 1.4** |
| PLGA 100/CS/Ins=250/180/40 μg/ml: | | |
| in the buffer of Table 2 | 195.6 ± 32.8 | + 33.3 ± 1.3 |
| in the buffer of Table 3 | 179.0 ± 35.9 | + 29.0 ± 1.3 |
| * measured in water | | |
| ** measured in 0.5 mM citric acid solution (pH 6.0) | | |
| CS: chitosan, Ins: insulin | | |

Example 2: Preparation of a small particle system surface-coated with drug-surface-coating polymer complex in a 20 mM MES buffer system with two different concentration of insulin

Insulin (pI about 5.3) was used as a protein drug, and chitosan was used as a positively charged surface-coating polymer.

Two ml of bovine insulin (Sigma, 160 μg/ml or 800 μg/ml) in 0.5 mM citric acid solution (pH 4.5) was added to 2 ml of chitosan (Bioneer 143 kDa, 0.72 mg/ml or 3.6 mg/ml) in 0.5 mM citric acid solution (pH 4.5) and the mixture was left at room temperature for at least about 30 min. Four ml of PLGA small particles (about 100 nm in diameter) suspension in 0.5 mM citric acid solution (pH 4.5; concentration of PLGA small particle: 0.5 mg/ml or 2.5 mg/ml) prepared as described in Preparation Example 1 was added to the chitosan/insulin solution and the mixture was left at room temperature for at least 1 hour. The pH was increased to 6.0 by adding NaOH (0.1-2.5N), and salts and supplements were added thereto to afford the same solvent composition of the suspension as that of the buffer described in Table 5.
Table 5

Composition of 20 mM MES 0.5 mM citric acid isotonic buffer (pH 6.0)

|  | MW | g/L | concentration (mM) | ion intensity (mM) |
| --- | --- | --- | --- | --- |
| D-glucose | 180.16 | 1.80 | 10 | ---- |
| $MgCl_2$ | 95.21 | 0.0468 | 0.492 | 1.476 |
| KCl | 74.55 | 0.340 | 4.56 | 4.56 |
| $CaCl_2 \cdot 2H_2O$ | 147.02 | 0.176 | 1.2 | 3.6 |
| citric acid | 192.1 | 0.096 | 0.5 | 0.8 |
| MES | 195.2 | 3.90 | 20 | 8 |
| NaCl | 58.44 | 7.92 | 135.56 | 135.56 |

The particle size and the zeta potential of the surface-coated small particles were measured by DLS 802 (Viscotek) and Zeta sizer 2000 (Malvern), respectively. The both surface-coated small particle samples showed a preferable particle size and zeta potential (Table 6); the particle size was about two-fold of that of the uncoated particles (Table 4) and the zeta potential was a highly positive potential. The both surface-coated small particles were found to be stable as colloidal suspensions.
Table 6

Surface-coated PLGA small particles at two different insulin concentrations

|  | diameter (nm) | zeta potential (mV) |
| --- | --- | --- |
| PLGA 100/CS/Ins = 250/180/40 μg/ml | 221.8 ± 77.9 | + 30.2 ± 2.5 |

(continued)

Surface-coated PLGA small particles at two different insulin concentrations

| | diameter (nm) | zeta potential (mV) |
|---|---|---|
| PLGR 100/CS/Ins = 1250/900/200 μg/ml | 189.6 ± 36.8 | + 30.2 ± 3.0 |
| CS: chitosan, Ins: insulin | | |

Example 3: Preparation of a surface-coated PLGA small particle system using poly-L-arginine as the surface-coating polymer

Insulin (about 5.3 in pI) was used as a protein drug, and poly-L-arginine was used as a positively charged surface-coating polymer.
3 ml of bovine insulin (Sigma, 40 μg/ml) in 0.5 mM citric acid solution (pH 6.0) was added to 3 ml of poly-L-arginine (MW 125 kDa, Sigma; 2.88 mg/ml) in 0.5 mM citric acid solution (pH 6.0) and the mixture was left at room temperature for at least about 30 min. 6 ml of PLGA small particles (about 100 nm in diameter) suspension in 0.5 mM citric acid solution (pH 6.0; concentration of PLGA small particle: 250 μg/ml) prepared as described in Preparation Example 1 was added to the poly-L-arginine/insulin solution and the mixture was left at room temperature for at least 1 hour. Salts and supplements were added thereto to afford the same solvent composition of the suspension as that of the buffer described in Table 5. The particle size and the zeta potential of the surface-coated small particles were measured by DLS 802 (Viscotek) and Zeta sizer 2000 (Malvern), respectively. The mean diameter of the particles was found to be 285.9 ± 90.6 nm, and the zeta potential was found to be + 48.3 ± 0.9 mV.

Example 4: Preparation of a surface-coated PLGA small particle system using chitosan and poly-L-arginine as the surface-coating polymers

Insulin (pI about 5.3) was used as a protein drug, and chitosan and poly-L-arginine were used as a positively charged surface-coating polymer.
2 ml of bovine insulin (Sigma, 800 μg/ml) in 0.5 mM citric acid solution (pH 4.5) was added to 2 ml of a mixture of chitosan (MW 143 kDa, 0.36 mg/ml) and poly-L-arginine (MW 125 kDa, Sigma; 1.8 mg/ml) in 0.5 mM citric acid solution (pH 4.5) and the mixture was left at room temperature for at least about 30 min. 4 ml of PLGA small particles (about 100 nm in diameter) suspension in 0.5 mM citric acid solution (pH 4.5; concentration of PLGA small particle: 2.5 mg/ml) prepared as described in Preparation Example 1 was added to the chitosan/poly-L-arginine/insulin solution and the mixture was left at room temperature for at least 1 hour. Salts and supplements were added thereto to afford the same solvent composition of the suspension as that of the buffer described in Table 7. The particle size and the zeta potential of the surface-coated small particles were measured by DLS 802 (Viscotek) and Zeta sizer 2000 (Malvern), respectively. The mean diameter of the particles was found to be 336.1 ± 20.8 nm, and the zeta potential was found to be + 40.3 ± 3.4 mV.
Table 7

Composition of 5 mM MES 0.5 mM citric acid isotonic buffer (pH 6.0)

| | MW | g/L | concentration (mM) | ion intensity (mM) |
|---|---|---|---|---|
| D-glucose | 180.16 | 1.80 | 10 | ---- |
| MgCl$_2$ | 95.21 | 0.0468 | 0.492 | 1.476 |
| KCl | 74.55 | 0.340 | 4.56 | 4.56 |
| CaCl$_2$·2H$_2$O | 147.02 | 0.176 | 1.2 | 3.6 |
| citric acid | 192.1 | 0.096 | 0.5 | 0.8 |
| MES | 195.2 | 0.98 | 5 | 2 |
| NaCl | 58.44 | 8.27 | 141.56 | 141.56 |

Example 5: Preparation of an insulin/chitosan surface-coated PLGA small particle system using another production method

3 ml of 0.1% w/v polystyrene small particles (MolecularProbe, carboxylated FluoSpheres) in a 0.5 mM citric acid solution (pH 4.5) was added to 3 ml of chitosan (Bioneer 143 kDa, 180 μg/ml) in 0.5 mM citric acid solution (pH 4.5) and the mixture was left at room temperature for at least 1 hour. 6 ml of bovine insulin (Sigma, 20 μg/ml) in 0.5 mM citric acid solution (pH 4.5) was added to the above-mentioned mixture of polystyrene and chitosan and the mixture was left at room temperature for at least 1 hour. The pH was increased to 6.0 by adding NaOH (0.1-2.5N), and salts and supplements were added thereto to afford the same solvent composition of the suspension as that of the buffer described in Table 5.

The particle size and the zeta potential of the surface-coated small particles were measured by DLS 802 (Viscotek) and Zeta sizer 2000 (Malvern), respectively. The mean diameter of the particles was found to be 302.0 ± 68.6 nm, and the zeta potential was found to be + 27.9 ± 1.7 mV. The diameter of the polystyrene core particles was 196.7 ± 27.5 nm, showing the presence of a layer with a thickness of about 50 nm around the periphery of the polystyrene core particles.

Example 6: Preparation of an insulin/chitosan surface-coated PLGA small particle system using another production method

1.8 ml of PLGA with a lactide:glycolide ratio of 50:50 (RESOMER RG 502H, Bohringer Ingelheim) in acetone solution (PLGA 0.01% (w/v): a concentration wherein PLGA particles of about 20 nm can be prepared) was added to 6 ml of chitosan (Bioneer 73 kDa, 0.25 mg/ml) in 0.5 mM citric acid solution (pH 4.5) and the mixture was left at room temperature until the acetone had fully evaporated. Three ml of bovine insulin (Sigma, 160 μg/ml) in 0.5 mM citric acid solution (pH 4.5) was added to 3 ml of the PLGA/chitosan suspension and the mixture was left at room temperature for at least 1 hour. The pH was increased to 6.0 by adding NaOH (0.1-2.5N), and salts and supplements were added to create the solvent composition of the suspension same as that of the buffer described in Table 2. The particle size of the surface-coated small particles was measured by DLS 802 (Viscotek). The mean diameter of the particles was found to be 146.1 ± 35.8 nm. The particles were stable as a colloidal suspension.

Example 7: Preparation of an insulin/chitosan surface-coated PLGA small particle system for animal testing

Insulin (about 5.3 in pI) was used as a protein drug, and chitosan was used as a positively charged surface-coating polymer. The sample was prepared to have a high concentration (insulin concentration 6 mg/mL) for an animal test. Bovine aqueous insulin solution (15 ml, Sigma, 320 μg/ml, pH 4.5) and 0.02 ml of 50 mM aqueous citric acid solution were added to 15 ml of aqueous chitosan solution (manufactured by Koyo Chemical, Koyo Chitosan FL-80, 1.44 mg/mL, pH 4.5), the pH was adjusted to 4.5 ± 0.1, and the mixture was left standing for about 1 hour. Then 30 ml of PLGA small particle (particle size about 100 nm, PLGA small particle concentration 1 mg/mL, pH 4.5) suspension and 0.02 ml of 50 mM aqueous citric acid solution were added, and the pH was adjusted to 4.5. The resultant solution was left standing for 1 hour, the pH was adjusted to 6.0, maltose (0.421 g) was dissolved therein, and the pH was confirmed to be 6. The thus-prepared solution was frozen with liquid nitrogen, and then freeze-dried. The freeze-dried product was dispersed again in distilled water in a volume equivalent to 1/15 of the solution before the freeze-drying. The re-suspension was centrifuged (19400xG, 3 hours, 4°C) and 4/5 volume of the supernatant was removed, whereby the particle fraction was concentrated to give a sample for an animal test (insulin concentration 6 mg/mL).

The particle size and the zeta potential of the surface-coated small particles were measured by Zeta sizer Nano (Malvern). The mean diameter of the particles was found to be 252 nm, and the zeta potential was found to be + 10.6 mV. The particles were found to be stable as colloidal suspensions. Additionally, the ratio of insulin bound to the surface-coated small particles in this Example was measured by the method described below to find a loading efficiency of 93%.

Example 8: Preparation of an insulin/chitosan surface-coated PLGA small particle system for release testing

Insulin (pI about 5.3) was used as a protein drug, and chitosan was used as a positively charged surface-coating polymer.

A bovine aqueous insulin solution (15 ml, Sigma, 320 μg/ml, pH 4.5) and 0.02 ml of 50 mM aqueous citric acid solution were added to 15 ml of aqueous chitosan solution (manufactured by Koyo Chemical, Koyo Chitosan FL-80, 1.44 mg/mL, pH 4.5), the pH was adjusted to 4.5 ± 0.1, and the mixture was left standing for about 1 hour. Then 30 ml of PLGA small particle (particle size about 100 nm, PLGA small particle concentration 1 mg/mL, pH 4.5) suspension and 0.02 ml of 50 mM aqueous citric acid solution were added, and the pH was adjusted to 4.5. The resultant solution was left standing for 1 hour, the pH was adjusted to 6.0, maltose (0.421 g) was dissolved therein, and the pH was confirmed to be 6.

Example 9: Preparation of a surface-coated PLGA small particle system (pH 8) using cationic chitosan derivative as the surface-coating polymer

Insulin (about 5.3 in pI) was used as a protein drug, and a cationic chitosan derivative was used as a positively charged surface-coating polymer.

Two ml of bovine insulin (Sigma, 0.32 mg/ml) in 0.5 mM citric acid solution (pH 4.5) was added to 2 ml of cationic chitosan derivative (manufactured by Dainichiseika, cationic chitosan derivative solution, 1.44 mg/ml) in 0.5 mM citric acid solution (pH 4.5), and the mixture was left standing at room temperature for at least 30 minutes. Four ml of a suspension of PLGA small particle (about 100 nm in diameter), which were prepared as mentioned in Preparation Example 1, in 0.5 mM citric acid (pH 4.5; PLGA small particle concentration: 1.0 mg/ml) was added to the cationic chitosan derivative/ insulin solution, and the mixture was left standing at room temperature for at least 1 hour. Maltose was added thereto to 10% w/v, and the pH was adjusted to 8 by adding NaOH.

The particle size of the surface-coated small particles was measured by Zeta sizer Nano (Malvern). The mean diameter of the particles was found to be 230 nm. The particles were found to be stable as colloidal suspensions. Additionally, the ratio of insulin bound to the surface-coated small particles in this Example was measured by the method described below to find a loading efficiency of 74% w/w.

Example 10: Preparation of a surface-coated PLGA small particle system (pH 7) using cationic chitosan derivative as the surface-coating polymer

Insulin (pI about 5.3) was used as a protein drug, and a cationic chitosan derivative was used as a positively charged surface-coating polymer.

Two ml of bovine insulin (Sigma, 0.32 mg/ml) in 0.5 mM citric acid solution (pH 7.0) was added to 2 ml of cationic chitosan derivative (manufactured by Dainichiseika, cationic chitosan derivative solution, 1.44 mg/ml) in 0.5 mM citric acid solution (pH 7.0), and the mixture was left at room temperature for at least 30 minutes. Four ml of a suspension of PLGA small particles (about 100 nm in diameter), which were prepared as mentioned in Preparation Example 1, in 0.5 mM citric acid (pH 7.0; PLGA small particle concentration: 1.0 mg/ml) was added to the cationic chitosan derivative/insulin solution, and the mixture was left standing at room temperature for at least 1 hour. Maltose was added thereto to 10% w/v, and the pH was confirmed to be 7.

The particle size and the zeta potential of the surface-coated small particles were measured by Zeta sizer Nano (Malvern). The mean diameter of the particles was found to be 234 nm, and the zeta potential was found to be + 11.3 mV. The particles were found to be stable as colloidal suspensions. Additionally, the amount of insulin bound to the surface-coated small particles in this Example was measured by the method described below to find a loading efficiency was found to be 65% w/w.

Example 11: Preparation of a surface-coated small particle system using a positively charged drug and a negatively charged surface-coating polymer

Zolmitriptan (pKa=9.5) was used as a positively charged low-molecular weight drug, and polyacrylic acid was used as a negatively charged surface-coating polymer.

Four ml of an aqueous suspension of trimethylamine-modified polystyrene particles (1 mg/ml, micromer NR3+ 100 nm, Corefront Corporation) was added to 2 ml of aqueous polyacrylic solution (manufactured by Wako Pure Chemical Industries, average molecular weight 250,000, 1.44 mg/ml), and the mixture was gently stirred. About 1 hour later, 2 ml of aqueous zolmitriptan solution (640 $\mu$g/ml) was added thereto, the mixture was gently stirred, and the pH was adjusted to 6.0.

The particle size and the zeta potential of the surface-coated small particles were measured by Zeta sizer Nano (Malvern). The mean diameter of the particles was found to be 330 nm, and the zeta potential was found to be - 75 mV. The particles were found to be stable as colloidal suspensions. Additionally, the amount of drug bound to the surface-coated small particles in this Example was measured according to the method described below (under different HPLC conditions) to find a loading efficiency of 14% w/w.

Example 12: Preparation of a surface-coated small particle system using a positively charged drug and a negatively charged surface-coating polymer

Bromhexin hydrochloride was used as a positively charged low-molecular weight drug, and sodium polyacrylate was used as a negatively charged surface-coating polymer.

One ml of aqueous bromhexin hydrochloride solution (640 $\mu$g/ml) and 1 ml of distilled water were added to 2 ml of aqueous sodium polyacrylate solution (degree of polymerization 2,700-7,500, manufactured by Wako Pure Chemical Industries, 1.44 mg/ml), and the mixture was gently stirred. About one hour later, 4 ml of an aqueous suspension of trimethylamine-modified polystyrene particles (1 mg/ml, micromer NR3+ 100nm, Corefront Corporation) was added thereto, the mixture was gently stirred, and the pH was adjusted to 6.

The particle size and the zeta potential of the surface-coated small particles were measured by Zeta sizer Nano (Malvern). The mean diameter of the particles was found to be 160 nm, and the zeta potential was found to be - 57 mV. The particles were found to be stable as colloidal suspensions. Additionally, the amount of drug bound to the surface-coated small particles in this Example was measured according to the method described below (under different HPLC conditions) to find a loading efficiency of 88% w/w.

Example 13: Preparation of a surface-coated small particle system using a positively charged drug and a negatively charged surface-coating polymer

Bromhexin hydrochloride was used as a positively charged low-molecular weight drug, and sodium poly-gamma-glutamate was used as a negatively charged surface-coating polymer.

One ml of aqueous bromhexin hydrochloride solution (640 $\mu$g/ml) and 1 ml of distilled water were added to 2 ml of sodium poly-gamma-glutamate solution (average molecular weight 200,000-500,000, manufactured by Wako Pure Chemical Industries, 1.44 mg/ml), and the mixture was gently stirred. About one hour later, 4 ml aqueous suspension of trimethylamine-modified.polystyrene particles (1 mg/ml, micromer NR3+ 100nm, Corefront Corporation) was added thereto, the mixture was gently stirred, and the pH was adjusted to 6.

The particle size and the zeta potential of the surface-coated small particles were measured by Zeta sizer Nano (Malvern). The mean diameter of the particles was found to be 203 nm, and the zeta potential was found to be - 63 mV. The particles were found to be stable as colloidal suspensions. Additionally, the amount of drug bound to the surface-coated small particles in this Example was measured according to the method described below (under different HPLC conditions) to find a loading efficiency of 32% w/w.

Experimental Example 1: Evaluation of interaction of drug and surface-coating polymer on the surface of small particles

One ml suspension of PLGA small particles surface-coated with insulin/chitosan (PLGA 100/chitosan/insulin=500/360/80 $\mu$g/ml) in the buffer described in Table 5 was added to a microtube and centrifuged at 18000 rpm (23900xg) for 60 min. The precipitate was rinsed with the buffer described in Table 5. After adding pH 4.5 or pH 6.0 release buffers to the precipitate, each tube was shaken at room temperature for 2 hour. The suspension was centrifuged again for 15 min under the same conditions and the supernatant collected. Then the insulin concentration in each supernatant buffer was quantified by ELISA.

It was shown that with the pH 4.5 buffer in which both chitosan and insulin carried positive charge and hence their attractive interaction due to electrostatic forces were low, the amount of insulin was remarkably higher than at ph 6.0 where the two were oppositely charged and hence the electrostatic attractive forces became greater (Fig. 2). It should be noted that free insulin and chitosan was removed by the above-mentioned wash operation prior to the release test. These results show the existence of a chitosan/insulin complex on the surface of the small particles.

One ml suspension of PLGA small particles surface-coated with insulin/chitosan (PLGA 100/chitosan/insulin=250/180/40 $\mu$g/ml) in the buffer described in Table 2 prepared by the method described in Example 1 was added to a microtube and centrifuged at 18000 rpm (23900xg) for 60 min. The precipitate was rinsed with the buffer described in Table 2. The precipitate after washing was resuspended in the buffer, spread on a glass plate and inartificially dried for about 48 hr to give a TOF-SIMS sample. As a control for comparison, glass plate samples with insulin, chitosan, or PLGA 100 prepared by the method of Preparation Example 1 spread thereon were also prepared.

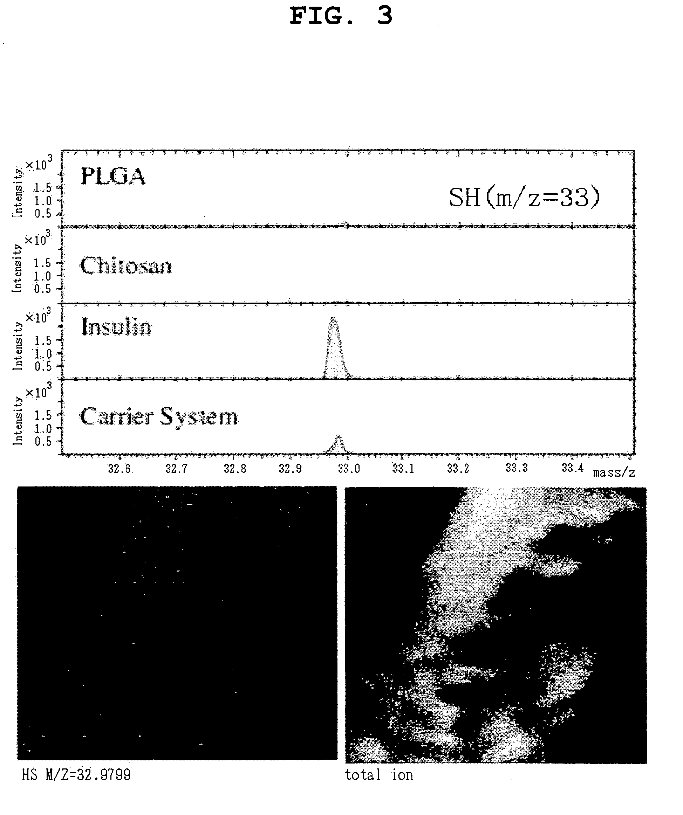

These samples were each measured by a TOF-SIMS IV apparatus (ION-TOF GmbH) for the presence or absence of a peak (m/z=33) corresponding to SH of cysteine residue of insulin. As a result, a significant level of m/z=33 peak was not detected in PLGA and chitosan. However, a strong peak was detected in the above-mentioned surface-coated small particle sample after washing and in the insulin sample. The results of the measurement for each sample are shown in the upper panel of Fig. 3.

In addition, m/z=33 distribution (lower left panel in Fig. 3) and total ion distribution (lower right panel in Fig. 3) in the observation field were also confirmed for the above-mentioned surface-coated small particles after washing, and it was also confirmed that the distribution patterns of both matched, namely, detection of m/z=33 from the particles.

The above-mentioned measurement results relating to the specificity and the results of particle distribution images indicate the presence of insulin on the surface of the surface-coated small particles after washing.

Experimental Example 2: Evaluation of drug loading efficiency and loading capacity

Samples to be subjected to the experiments were prepared as follows.

[PLGA/chitosan/insulin (1000/720/160 $\mu$g/ml) surface coated small particle (in a buffer described in Table 8)]

Insulin (pl about 5.3) was used as a protein drug, and chitosan was used as a positively charged surface-coating polymer.

4.5 ml of bovine insulin (Sigma, 0.64 mg/ml) in 0.5 mM citric acid solution (pH 4.5) was added to 4.5 ml of chitosan (Bioneer 143 kDa, 2.88 mg/ml) in 0.5 mM citric acid solution (pH 4.5) and the mixture was left at room temperature for at least about 30 min. Nine ml of PLGA small particles (about 100 nm in diameter) suspension in 0.5 mM citric acid solution (pH 4.5; concentration of PLGA small particle: 2.0 mg/ml) prepared as described in Preparation Example 1 was added to the chitosan/insulin solution and the mixture was left at room temperature for at least 1 hour. The pH was increased to 6.0 by adding NaOH (0.1-2.5N), and glucose was added thereto to afford the same solvent composition of the suspension as that of the buffer described in Table 8.

Table 8

Composition of 100 mM glucose 0.5 mM citrate buffer (pH 6.0; non-isotonic)

|  | MW | g/L | concentration (mM) | ion intensity (mM) |
|---|---|---|---|---|
| D-glucose | 180.16 | 18.0 | 100 | ---- |
| citric acid | 192.1 | 0.096 | 0.5 | 0.8 |

The particle size and the zeta potential of the surface-coated small particles were measured by DLS 802 (Viscotek) and Zeta sizer 2000 (Malvern), respectively. The mean diameter of the particles was found to be 248.8 $\pm$ 94.2 nm, and the zeta potential was found to be + 8.7 $\pm$ 0.5 mV.

[PLGA/chitosan/insulin (250/90/40 $\mu$g/ml) surface coated small particles (in the buffer described in Table 8)]

Insulin (pI about 5.3) was used as a protein drug, and chitosan was used as a positively charged surface-coating polymer.

Three ml of bovine insulin (Sigma, 160 $\mu$g/ml) in 0.5 mM citric acid solution (pH 4.5) was added to 3 ml of chitosan (Bioneer 143 kDa, 0.36 mg/ml) in 0.5 mM citric acid solution (pH 4.5) and the mixture was left at room temperature for at least about 30 min. Six ml of PLGA small particles (about 100 nm in diameter) suspension in 0.5 mM citric acid solution (pH 4.5; concentration of PLGA small particle: 500 $\mu$g/ml) prepared as described in Preparation Example 1 was added to the chitosan/insulin solution and the mixture was left at room temperature for at least 1 hour. The pH was increased to 6.0 by adding NaOH (0.1-2.5N), and glucose was added thereto to afford the same solvent composition of the suspension as that of the buffer described in Table 8.

The particle size and the zeta potential of the surface-coated small particles were measured by DLS 802 (Viscotek) and Zeta sizer 2000 (Malvern), respectively. The mean diameter of the particles was found to be 253.7 $\pm$ 31.3 nm, and the zeta potential was found to be + 6.4 $\pm$ 1.8 mV.

[PLGA/poly-L-arginine/insulin (125/720/40 $\mu$g/ml) surface coated small particle (in the buffer described in Table 5)]

Insulin (pI about 5.3) was used as a protein drug, and poly-L-arginine was used as a positively charged surface-coating polymer.

Three ml of bovine insulin (Sigma, 160 $\mu$g/ml) in 0.5 mM citric acid solution (pH 6.0) was added to 3 ml of poly-L-arginine (MW 125 kDa, Sigma; 2.88 mg/ml) in 0.5 mM citric acid solution (pH 6.0) and the mixture was left at room temperature for at least about 30 min. Six ml of PLGA small particles (about 100 nm in diameter) suspension in 0.5 mM citric acid solution (pH 6.0; concentration of PLGA small particle: 250 $\mu$g/ml) prepared as described in Preparation Example 1 was added to the poly-L-arginine/insulin solution and the mixture was left at room temperature for at least 1 hour. Salts and supplements were added thereto to afford the same solvent composition of the suspension as that of the buffer described in Table 5. The particle size and the zeta potential of the surface-coated small particles were measured by DLS 802 (Viscotek) and Zeta sizer 2000 (Malvern), respectively. The mean diameter of the particles was found to be 497.4 $\pm$ 141.9 nm, and the zeta potential was found to be + 44.3 $\pm$ 2.1 mV.

The loading efficiency and loading capacity of insulin was measured by the method described below.

[Analytical method of bound insulin]

0.5 ml or 1 ml of each of the above-mentioned samples was added into 1.5 ml microtube and centrifuged (15000 rpm (21900xg), 180 min, 4°C). The supernatant was collected and the insulin concentration in the supernatant was measured using an insulin ELISA kit (Mercodia Bovine insulin ELISA) and a dilution buffer (Mercodia Diabetes sample buffer) or HPLC (the insulin concentration is taken as A). As a control, 0.5 ml or 1 ml of each of the above-mentioned samples in 1.5 ml microtube was kept in 4°C for 180 min and the insulin concentration in all of the samples was measured in the same way (the insulin concentration is taken as B).

Loading efficiency and loading capacity are calculated as follows:

```
Loading efficiency (%) = 100×((mean value of B)-A)/(mean value of
B));
```

```
Loading capacity (%) = mass of insulin bound to core particle
total mass
           =100×((mean value of B)-A)/core particle total mass
(core particle total mass=number of particles×4/3×3.14×(core
```
$$\text{particle radius})^3 \times \text{density}).$$

The analysis results of the insulin loading by the above-mentioned method are shown in Table 9.

Table 9

Insulin loading efficiency and insulin loading capacity of surface-coated small particle

|  | a (%) | b (%) |
|---|---|---|
| PLGA/CS/Ins=1000/720/160 $\mu$g/ml (in the buffer described in Table 8) | 84.7 ± 0.3 | 12.4 ± 0.1 |
| PLGA/CS/Ins=250/90/40 $\mu$g/ml (in the buffer described in Table 8) | 44.0 ± 2.9 | 6.4 ± 0.4 |
| PLGA/Poly-L-Arginine/Ins=125/720/40 $\mu$g/ml (in the buffer described in Table 5) | 57.2 ± 1.0 | 17.7 ± 0.3 |

a: loading efficiency
b: loading capacity
CS: chitosan, Ins: insulin

Experimental Example 3: Evaluation of stability of insulin complexed on surface of small particles

PLGA/chitosan/insulin (1000/720/160 $\mu$g/ml) surface coated small particles in the buffer described in Table 8, which were used in Experimental Example 2, were used as the sample in the present experiment. As a control, insulin solution with the same solvent composition as that of the buffer described in Table 8 (pH 6; insulin concentration: 160 $\mu$g/ml) was used.

The sample to be used for the enzymatic reaction was prepared as follows.
$\alpha$-chymotrypsin derived from bovine pancreas (Fluka Biochemika; code No. 27270) was dissolved in 5 mM MES buffer (pH 6.0) to a concentration of 40 $\mu$g/ml. 1 ml of sample was warmed at 37°C for 15 min, and 0.6 ml of 5 mM MES buffer (pH 6.0) pre-warmed in the same manner and 0.4 ml of $\alpha$-chymotrypsin in 5 mM MES buffer (pH 6.0; $\alpha$-chymotrypsin concentration: 40 $\mu$g/ml) pre-warmed in the same manner were added. The mixture was incubated with shaking at 37°C for 30 min. The enzymatic reaction was stopped by adding 1 ml of ice-cooled acetic acid glacial. The reaction mixture was stirred in a mixer for 1 min, left at room temperature for not less than 1 hour, and passed through a filter with 0.1 $\mu$m filter diameter to give sample for HPLC analysis.

The control sample containing no enzymes was prepared as follows.
1 ml of sample was mixed with 1 ml of 5 mM MES buffer (pH 6.0) and 1 ml of acetic acid glacial. Then the mixture was stirred in a mixer for 1 min, left at room temperature for not less than 1 hour, and passed through a filter with 0.1 $\mu$m filter diameter to give sample for HPLC analysis.

The standard sample for the calibration curve for the insulin quantitation was prepared as follows.
1 ml of insulin solution (40-160 $\mu$g/ml) was mixed with 1 ml of 5 mM MES buffer (pH 6.0) and 1 ml of acetic acid glacial, and the mixture was stirred in a mixer. The mixture was used as standard sample for calibration curve of HPLC analysis.

HPLC analysis of the samples was performed under the following conditions:

C18 column (Inertsil ODS-2, 5 $\mu$m, 250 mm × 4.6 mm) ;

mobile phase A: 0.1% TFA aqueous solution, mobile phase B: 0.1% TFA $CH_3CN$ solution;
gradient conditions (mobile phase B concentration): at 0 min: 30%, at 10 min: 40%, at 11 min: 30%, at 16 min: 30%;
column oven temperature: 40°C, flow rate: 1.0 ml/min, injection volume: 20 $\mu$l, detection: UV275nm.

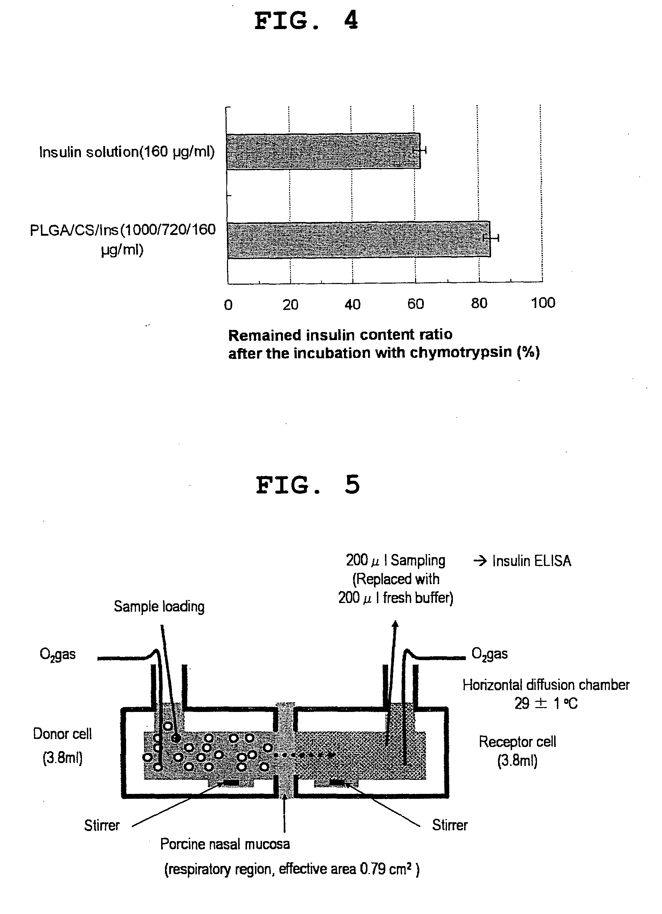

As a result of the analysis under the above-mentioned conditions, the proportion of insulin remaining after the enzymatic reaction was 83.9% $\pm$ 2.3% in PLGA/chitosan/insulin and 61.8% $\pm$ 2.0% in the insulin solution. In other words, only 16.1% of the surface coated complexed insulin was degraded during the time of the experiment compared to 38.2% for the free insulin solution (Fig. 4).
These results show marked improvement in the stability of insulin against the enzymatic reaction.

Experimental Example 4: Evaluation of insulin transport across porcine nasal mucosa using surface-coated small particle

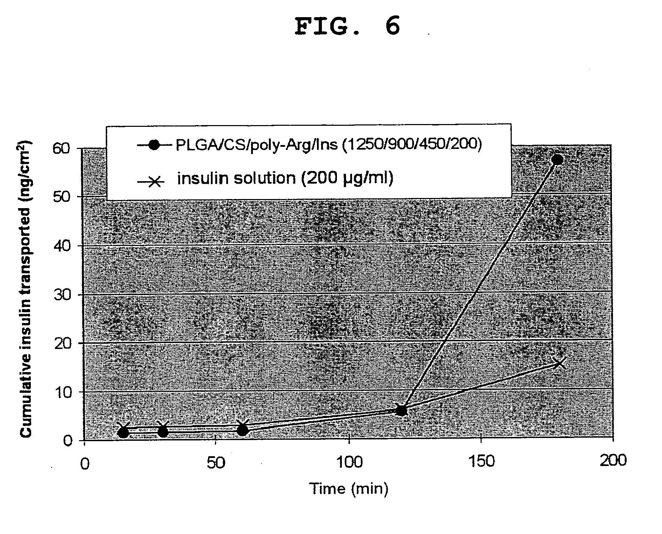

Insulin (pI about 5.3) was used as a protein drug, and chitosan and poly-L-arginine were used as positively charged surface-coating polymers. The surface-coated small particles were prepared in the same manner as in Example 4. As a control, an insulin solution with the same solvent composition as the buffer described in Table 7 (pH 6; insulin concentration: 200 $\mu$g/ml) was used. Nasal respiratory mucosal tissue was isolated from the porcine nasal cavity (respiratory region). The isolated tissue was preserved in a buffer with the same composition as that of the buffer used in Example 4 (oxygenated and cooled) before mounting on a horizontal diffusion chamber. The tissue was cut into a suitable size and mounted between the donor cell and the receptor cell in the horizontal diffusion chamber as shown in Fig. 5 (effective area of the mucosa: 0.79 $cm^2$). Fresh (above-mentioned) buffer (oxygenated and cooled) was injected into the donor cell and receptor cell, and the both cells were incubated in a circulation water heated to 29 $\pm$ 1°C for 30 min. and the tissue was equilibrated. The buffer injected into the donor cell and receptor cell was treated with oxygen during the transport study, and the both cells were warmed with circulation water heated to 29 $\pm$ 1°C. The viability of the tissue before and after the transport study and the absence of damage was confirmed by Alamar Blue Assay and the measurement of the TEER value of the tissue.
The transport study was started by replacing the whole buffer in the donor cell with the same amount of each sample. 200 $\mu$l of sample was taken from the receptor cell at selected time points and replaced with the same amount of fresh buffer (oxygenated and heated to 29 $\pm$ 1°C). Samples taken from the receptor cell were analyzed using an insulin ELISA kit and dilution buffer. The results of the analysis are shown in Fig. 6. Fig. 6 indicates that a greater amount of insulin was transported through isolated nasal respiratory mucosal tissue when chitosan/poly-L-arginine/insulin surface-coated PLGA small particles were added, compared to the addition of control insulin solution.

Experimental Example 5: Measurement of particle size distribution of surface-coated small particles

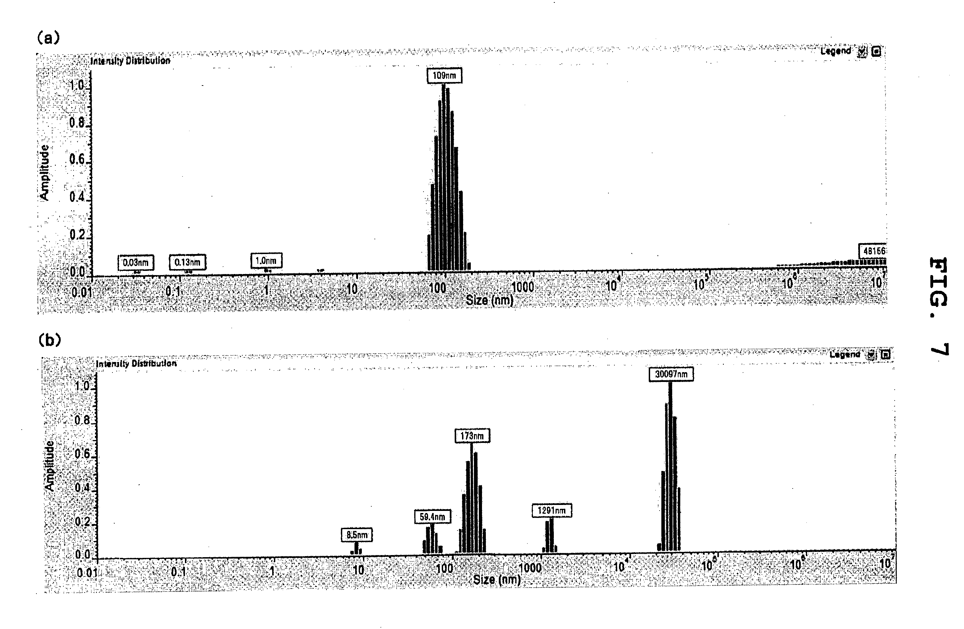

Insulin (pI about 5.3) was used as a protein drug, and chitosan was used as a positively charged surface-coating polymer.
1.5 ml of bovine insulin (Sigma, 0.8 mg/ml) in 0.5 mM citric acid solution (pH 4.5) was added to 1.5 ml of chitosan (Bioneer 143 kDa, 3.6 mg/ml) in 0.5 mM citric acid solution (pH 4.5) and the mixture was left at room temperature for at least 30 min. 3 ml of PLGA small particles (about 100 nm in diameter; hereinafter, also to be referred as "PLGA 100") suspension in 0.5 mM citric acid solution (pH 4.5; concentration of PLGA small particle: 2.5 mg/ml) prepared as described in Preparation Example 1, or a simple 0.5 mM citric acid solution (pH 4.5) was added to the chitosan/insulin solution and the mixture was left at room temperature for at least 1 hour. The pH was increased to 6.0 by adding NaOH (0.1-2.5N), and salts and supplements were added thereto to afford the same solvent composition of the suspension as that of the buffer described in Table 2. The particle size of the surface-coated small particles or chitosan/insulin mixture was measured by DLS 802 (Viscotek).
The results are shown in Fig. 7. Fig. 7(a) shows particle size distribution of surface-coated small particle, and Fig. 7(b) shows particle size distribution of chitosan/insulin mixture. In addition, the intensity at peak region, percentage, particle size and standard deviation of each of them are shown in Tables 10 and 11.
[Table 10]

| % in region | diameter (nm) | standard deviation |
|---|---|---|
| 0.2 | 0.1 | - |
| 0.2 | 0.3 | 0.0 |
| 0.3 | 2.0 | 0.1 |
| 93.3 | 218.3 | 51.6 |

(continued)

| % in region | diameter (nm) | standard deviation |
|---|---|---|
| 6.0 | 9.6E+06 | 5.3E+06 |

[Table 11]

| % in region | diameter (nm) | standard deviation |
|---|---|---|
| 1.4 | 17.1 | 1.1 |
| 7.4 | 118.9 | 14.0 |
| 37.8 | 345.9 | 54.9 |
| 5.8 | 2.6E+03 | 1.9E+02 |
| 47.6 | 6.0E+04 | 7.6E+03 |

When PLGA particles were added, a monodispersed particle size was obtained. When PLGA particles were not added, a multidispersed particle size distribution including large particle size was obtained. The results reveal that the presence of core particles affords surface-coated small particles with uniform particle size.

Experimental Example 6: Confirmation of possibility of formulating a surface-coated small particle suspension as a dry powder

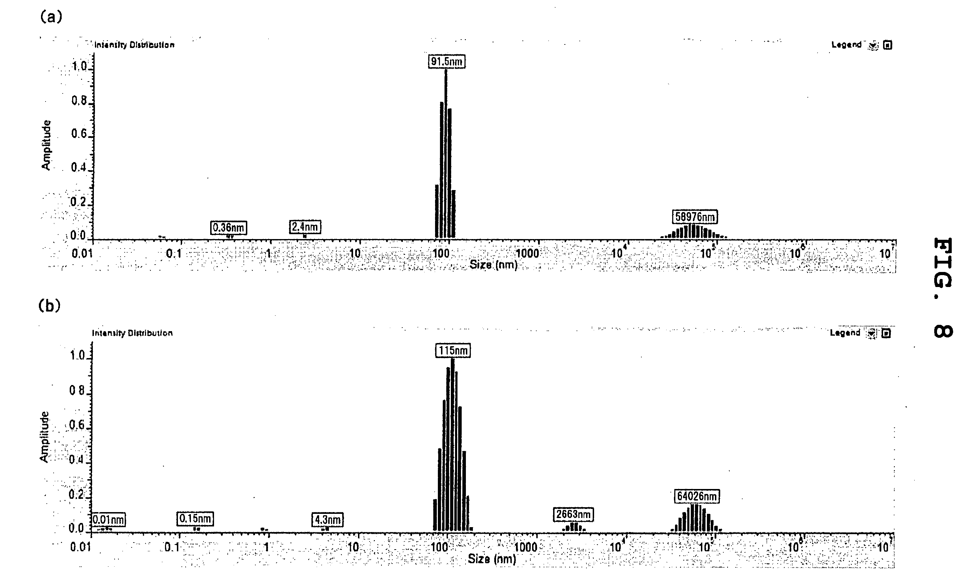

[PLGA100/chitosan/insulin (250/180/40 $\mu$g/ml) surface coated small particles (in the buffer described in Table 2)] were prepared by the method of Example 1. This suspension was freeze-dried and resuspended in an equal amount of water as before the freeze-drying. The particle size before the freeze-drying and the particle size after the freeze-drying and resuspension were measured using a DLS 802 (Viscotek). The results of particle size measurements are shown in Fig. 8. Fig. 8(a) shows particle size distribution before freeze-drying and Fig. 8(b) shows particle size distribution after freeze-drying and resuspending. In addition, the intensity at peak region, percentage, particle size and standard deviation of each of them are shown Tables 12 and 13.
[Table 12]

| % in region | diameter (nm) | standard deviation |
|---|---|---|
| 0.3 | 0.7 | 0.0 |
| 0.3 | 4.9 | 0.0 |
| 81.7 | 183.0 | 21.2 |
| 17.6 | 118,000.0 | 40,000.0 |

[Table 13]

| % in region | diameter (nm) | standard deviation |
|---|---|---|
| 0.4 | 0.0 | 0.0 |
| 0.4 | 0.3 | 0.0 |
| 0.3 | 8.7 | 0.4 |
| 80.6 | 229.4 | 47.9 |
| 2.3 | 5,325.6 | 669.2 |
| 15.9 | 128,000.0 | 34,200.0 |

The particle size before freeze-drying was 183.0 $\pm$ 21.2 nm, and the particle size after freeze-drying and resuspending was 229.4 $\pm$ 47.9 nm. The results show that the particle size of the main component did not change significantly due to freeze-drying and resuspending, and conspicuous aggregates were not produced. From such results, it is clear that the surface-coated small particles of the present invention can be used not only as a suspension but also in other dosage forms such as dry powder and the like.

Experimental Example 7: test of in vivo administration of surface-coated small particles to the rat nasal cavity

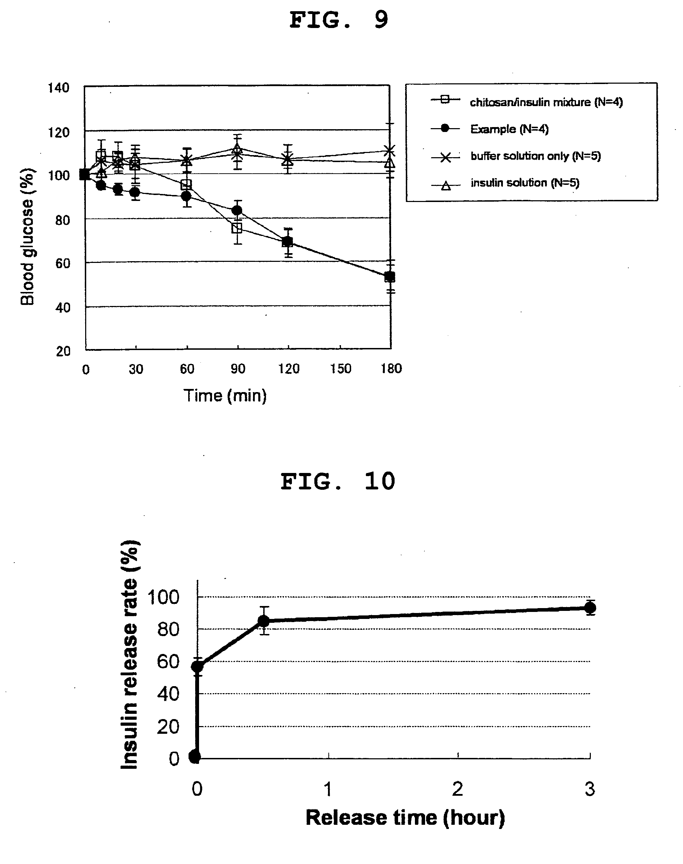

Rats (lineage: SD rat, 7 weeks old, male, breeder: Japan SLC) were used for the test. The rats were fasted from the evening of one day before the test. The test was performed under anesthesia by intramuscular injection and abdominal infusion. After the blood glucose concentration became stable, the sample was administered into the nasal cavity, and the blood was sampled from the tail vein at 10, 20, 30, 60, 90 and 120 minutes later. The blood glucose level was measured using a blood glucose detection kit (manufactured by Terumo, Medisafe Mini).
As the sample, the surface-coated small particles described in Example 7 were administered into the nasal cavity of rats at 20 $\mu$l/300 g body weight (400 $\mu$g insulin/kg body weight). As a control, 10% w/v maltose solution in 0.5 mM citric acid (pH 6) (buffer solution), insulin solution (pH 6: insulin concentration: 6 mg/ml, 10% w/v maltose in 0.5 mM citric acid), chitosan/insulin mixture (pH 6; chitosan concentration: 27 mg/ml, insulin concentration: 6 mg/ml, 10% w/v maltose in 0.5 mM citric acid), each having the same solvent composition as in Example 7, were also administered into the nasal cavity of rats.
The test results are shown in Fig. 9. Fig. 9 reveals that the blood glucose level decreased drastically as compared to the administration of control buffer solution and insulin solution and the initial blood glucose level decreased rapidly even when compared to a chitosan/insulin mixture, and that the Examples of the present invention exhibit a superior promoting effect on the transmucosal delivery of peptide drugs.

Experimental Example 8: In vitro release test using surface-coated small particles

0.5 mL of the sample from Example 8 was added into a 1.5 mL Eppendorf tube and centrifuged (13600 rpm (19400$\times$G), 3 hr, 4°C) to give precipitates. As a liquid for the release test, 152 mM aqueous NaCl solution in 5 mM MES was prepared (pH 6.0, physiologically isotonic ionic strength 154 mM).
0.5 mL of the liquid for the release test was added to the precipitate, and the precipitate was redispersed by pipetting and in a mixer and the dispersion was placed in a 10 mL glass vial. The Eppendorf tube was washed with 0.5 mL of fresh liquid for the release test, and the washing solution was also placed in the above-mentioned glass vial (total suspension 1 mL). The glass vial containing the suspension was shaken at 37°C, 75 rpm to perform a release test. After a predetermined time (0, 0.5 and 3 hours), the suspension was passed through a 0.1 $\mu$m filter (Sartorius) to give a filtrate. The filtrate was mixed with a 1/2 volume of 5% phosphoric acid in a mixer to give an HPLC sample for analysis of the amount of released insulin.
To quantify the insulin content before release, 1 mL of distilled water and 0.5 mL of 5% phosphoric acid were added to the precipitate, mixed in a mixer and left standing overnight at 4°C. The mixture was mixed again in a mixer and passed through a 0.1 $\mu$m filter (Sartorius) to give an HPLC sample for quantifying insulin content in the precipitate.
Insulin was quantified using the aforementioned insulin HPLC analysis conditions.
Release rate (%) = 100 $\times$ (insulin content of released liquid/insulin content of precipitate before release)
The results of the release test are shown in Fig. 10, wherein almost all of insulin contained in the precipitate was released within 3 hours of the release test.

**Industrial Applicability**

The use of the composition of the present invention enables an efficient transmucosal administration of low-molecular weight drugs and polymeric drugs such as peptides and proteins, which have so far been difficult to administer by a method other than injection. The drug contained in the composition of the present invention forms a complex with a surface-coating polymer and a small particle and thereby has higher stability (e.g., stability against enzymes, preservation stability) than when contained in a solution preparation, as well as a higher drug loading capacity compared to small particle preparations wherein a drug is encapsulated in a matrix of the small particle. Furthermore, it is possible to create surface-coated small particles that show sustained release or immediate release of the drug and control transmucosal absorbability of the drug according to the kind of the surface-coating polymer that forms a complex with the drug on the surface of the small particle.
This application is based on patent application No. 2008-172669 (filing date: July 1, 2008) filed in Japan, the contents of which are entirely incorporated herein.

**Claims**

1. A pharmaceutical composition for transmucosal administration, comprising (a) a drug having a positive or negative charge at a predetermined pH, (b) a pharmaceutically acceptable small particle and (c) a pharmaceutically acceptable surface-coating polymer capable of being electrically charged at the pH, wherein the surface of the small particle is coated by the surface-coating polymer, the drug is immobilized on the surface of the small particle via the surface-coating polymer, and a complex is formed by a noncovalent interaction between the small particle and the surface-coating polymer and a concurrent electrostatic interaction between the surface-coating polymer and the drug.

2. The composition of claim 1, wherein the noncovalent interaction between the small particle and the surface-coating polymer is electrostatic interaction.

3. The composition of claim 1 or 2, wherein the predetermined pH is the physiological pH of an administration site.

4. The composition of any one of claims 1 to 3, wherein the drug is selected from the group consisting of peptide, protein, DNA, RNA, siRNA, polysaccharide, antigen and low-molecular weight drug.

5. The composition of any one of claims 1 to 4, wherein the drug is a drug capable of producing medicinal or vaccine effect.

6. The composition of claim 4, wherein the drug is insulin.

7. The composition of claim 4, wherein the drug is at least one drug selected from the group consisting of bromhexine, zolmitriptan and salts thereof.

8. The composition of any one of claims 1 to 7, wherein the surface-coating polymer is by itself slightly water-soluble at the predetermined pH.

9. The composition of any one of claims 1 to 8, wherein the surface-coating polymer is at least one polymer selected from the group consisting of chitosan, polyarginine, polyacrylic acid, poly-gamma-glutamic acid and salts thereof.

10. The composition of any one of claims 1 to 9, wherein the surface-coating polymer is mucoadhesive and/or acts as a transmucosal absorption promoter.

11. The composition of any one of claims 1 to 10, wherein the small particle comprises a polymer having a carboxylic or an amino group.

12. The composition of any one of claims 1 to 11, wherein the small particle is comprised of a poly(lactic acid-glycol acid) copolymer.

13. The composition of any one of claims 1 to 12, wherein the mean particle size of the complex at the predetermined pH is not less than 10 nm and not more than 50 $\mu$m.

14. A production method of the composition of claim 8, comprising

(a) mixing the drug, the small particle and the surface-coating polymer at a pH at which the surface-coating polymer is readily water-soluble, and
(b) adjusting the pH of the mixture to the predetermined pH.

15. A production method of the composition of any one of claims 1 to 13, comprising

(a) mixing the drug, the surface-coating polymer and the small particle under a pH condition under which the drug and the surface-coating polymer have the same sign of the charge, and then
(b) adjusting the pH of the mixture to a pH at which the sign of the charge of the drug changes to the opposite sign, and
wherein the drug is an amphoteric drug.

16. The production method of claim 15, wherein the small particle has a charge of the sign opposite to that of the charge of the drug and the charge of the surface-coating polymer under the pH condition of step (a).

**17.** A production method of the composition of any one of claims 1 to 13, comprising

(a) adding dropwise an organic solvent solution of a material of the small particle into an aqueous solution of the surface-coating polymer,
(b) evaporating the organic solvent,
(c) adding the drug and stirring the mixture, and
(d) adjusting the pH of the mixture to the predetermined pH.

FIG. 1

FIG. 2

# FIG. 3

HS M/Z=32.9799          total ion

# FIG. 4

Insulin solution(160 µg/ml)

PLGA/CS/Ins(1000/720/160 µg/ml)

0    20    40    60    80    100

**Remained insulin content ratio
after the incubation with chymotrypsin (%)**

# FIG. 5

200 µ l Sampling    → Insulin ELISA
(Replaced with
200 µ l fresh buffer)

Sample loading

O₂gas

O₂gas

Horizontal diffusion chamber
29 ± 1 °C

Donor cell
(3.8ml)

Receptor cell
(3.8ml)

Stirrer

Stirrer

Porcine nasal mucosa
(respiratory region, effective area 0.79 cm² )

# FIG. 6

# FIG. 7

FIG. 8

# FIG. 9

Legend:
- □ chitosan/insulin mixture (N=4)
- ● Example (N=4)
- ✕ buffer solution only (N=5)
- △ insulin solution (N=5)

X-axis: Time (min)
Y-axis: Blood glucose (%)

# FIG. 10

X-axis: Release time (hour)
Y-axis: Insulin release rate (%)

<table>
<tr><td colspan="2" align="center">**INTERNATIONAL SEARCH REPORT**</td><td>International application No.<br>PCT/JP2009/062053</td></tr>
</table>

A. CLASSIFICATION OF SUBJECT MATTER
See extra sheet.


According to International Patent Classification (IPC) or to both national classification and IPC

B. FIELDS SEARCHED

Minimum documentation searched (classification system followed by classification symbols)
A61K9/16, A61K31/137, A61K31/4045, A61K31/715, A61K38/00, A61K38/28, A61K39/00, A61K45/00, A61K47/30, A61K47/32, A61K47/36, A61K47/42, A61K48/00, A61P3/10, A61P11/10, A61P25/06

Documentation searched other than minimum documentation to the extent that such documents are included in the fields searched
Jitsuyo Shinan Koho         1922-1996    Jitsuyo Shinan Toroku Koho    1996-2009
Kokai Jitsuyo Shinan Koho   1971-2009    Toroku Jitsuyo Shinan Koho    1994-2009

Electronic data base consulted during the international search (name of data base and, where practicable, search terms used)


C. DOCUMENTS CONSIDERED TO BE RELEVANT

| Category* | Citation of document, with indication, where appropriate, of the relevant passages | Relevant to claim No. |
|---|---|---|
| X<br>A | JP 2005-531487 A (Isis Pharmaceuticals Inc.),<br>20 October, 2005 (20.10.05),<br>Claims 1, 2, 10; Par. Nos. [0015], [0030] to<br>[0034]; example 3<br>& US 2003/0083286 A1    & EP 1427431 A<br>& WO 2003/018134 A2 | 1-13<br>14-17 |
| X<br>A | WO 2007/076295 A2 (UNIVERSITY OF KANSAS),<br>05 July, 2007 (05.07.07),<br>Claims 1 to 7, 12, 15 to 20, 22 to 25, 28 to 33,<br>43, 46; Par. Nos. [0009], [0011], [0050],<br>[0053], [0054], [0056] to [0067]; examples 2, 3<br>& JP 2009-519975 A    & US 2007/0172653 A1<br>& US 2008/0075667 A1    & US 2009/0053316 A<br>& US 2009/0081295 A    & EP 1968550 A | 1-13<br>14-17 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>22 July, 2009 (22.07.09) | Date of mailing of the international search report<br>04 August, 2009 (04.08.09) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/062053 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2005/089926 A1  (Kyowa Hakko Kogyo Co., Ltd.), 29 September, 2005 (29.09.05), & US 2008/0226704 A1    & EP 1728550 A1 | 1-17 |

Form PCT/ISA/210 (continuation of second sheet) (April 2007)

| INTERNATIONAL SEARCH REPORT | International application No. |
|---|---|
| | PCT/JP2009/062053 |

Continuation of A. CLASSIFICATION OF SUBJECT MATTER
 (International Patent Classification (IPC))

A61K9/16(2006.01)i, A61K31/137(2006.01)i, A61K31/4045(2006.01)i,
A61K31/715(2006.01)i, A61K38/00(2006.01)i, A61K38/28(2006.01)i,
A61K39/00(2006.01)i, A61K45/00(2006.01)i, A61K47/30(2006.01)i,
A61K47/32(2006.01)i, A61K47/36(2006.01)i, A61K47/42(2006.01)i,
A61K48/00(2006.01)i, A61P3/10(2006.01)i, A61P11/10(2006.01)i,
A61P25/06(2006.01)i

                    (According to International Patent Classification (IPC) or to both national
                    classification and IPC)

Form PCT/ISA/210 (extra sheet) (April 2007)

## INTERNATIONAL SEARCH REPORT

| International application No. |
| --- |
| PCT/JP2009/062053 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
    The invention of claim 1 is not novel, as disclosed in Documents 1 and 2.  Therefore, the invention has no special technical feature.

    Document 1: JP 2005-531487 A
    Document 2: WO 2007/076295 A2

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008172669 A

**Non-patent literature cited in the description**

- *Drug Discovery Today,* 2002, vol. 7, 1184-1189
- *J. Control. Rel.,* 2003, vol. 87, 187-198
- *J. Pharm. Sci.,* 2007, vol. 96, 473-483
- *Biomaterials,* 2002, vol. 23, 3193-3201
- *Int. J. Pharm.,* 2007, vol. 342, 240-249
- **Champion JA. et al.** *Proc. Natl. Acad. Sci. USA,* 2007, vol. 104, 11901-4
- **Chattopadhyay P. et al.** *Adv. Drug Deliv. Rev.,* 2007, vol. 59, 443-53
- **Zhou WY et al.** *J. Mater. Sci. Mater. Med.,* 2008, vol. 19, 103-110
- **Schaffazick SR et al.** *Pharmazie,* 2007, vol. 62, 354-60
- **Almeida AJ et al.** *Adv. Drug Deliv. Rev.,* 2007, vol. 59, 478-90
- **Muller, R.H.** Colloidal Carriers for Controlled Drug Delivery and Targeting: Modification, Characterization and In vivo Distribution. CRC Press, 1991
- Colloidal Drug Delivery Systems. Marcel Dekker, 1994